# EUROPEAN PATENT APPLICATION

(11) **EP 4 822 611 A1**
(43) Date of publication of application: **14.10.2026**
(21) Application number: 26171481.0
(22) Date of filing: 09.04.2026
(51) Int. Cl.: G06N 10/60, G16C 10/00

(54) **SYSTEM AND METHOD FOR DETERMINING OR MEASURING A SPECTRUM OF ONE OR MORE MOLECULE USING A QUANTUM COMPUTER**

(30) Priority: 10.04.2025 US 202563786470 P
(71) Applicant: Xanadu Quantum Technologies Inc., Toronto, ON M5G 2C8 (CA)
(72) Inventor: LOAIZA GANEM, Ignacio, Toronto, M6P 2R7 (CA); FOMICHEV, Stepan, Toronto, M5A 0C4 (CA); STETINA, Torin Fields, Seattle, WA 98103 (US); KUNITSA, Aleksandr, Newton, MA 02465 (US); MORENO CASARES, Pablo Antonio, 06011 Badajoz (ES); MOTLAGH HAGH NEGAHDAR, Danial, Toronto, M4Y 0E9 (CA)
(74) Representative: Hoyng Rokh Monegier B.V.

(57) **Abstract**

The method can include computing a leading-order error operator based on fragments of a Hamiltonian representing the one more molecules, where the fragments can be diagonalized, and a product formula approximating the time evolution; computing a set of eigenstates representing a classical solution of the Hamiltonian; computing product formula expectation values of the leading-order error operator for at least some eigenstates of the set; computing a maximum time step associated to a target error using the product formula expectation values; and performing a quantum phase estimation quantum computing process to determine the spectrum, including performing one or more Fourier transforms of the product of the distribution with the one or more expectation values in a discretized manner, over one or more time steps, the one or more time steps being equal to or lesser than the maximum time step.

## Description

### FIELD

This specification relates to quantum sensing, and more specifically to first-order dipole spectroscopy.

### BACKGROUND

Significant research and development efforts have been made in recent years to harness the quantum properties of quantum systems of matter for human use, and the related technologies are expected to become more mainstream within the coming years. A lot of this new interest is tied to the immense potential of these technologies, particularly in relation to quantum computing and computerized communication security, but also in relation to other significant areas of interest such as quantum sensing.

For instance, measuring the spectrum of one or more molecules following an electromagnetic interaction, such as with light, protons or electrons, can be useful for applications of quantum sensing. Indeed, spectroscopy can be used to compare a spectrum of a sample (which can be enclosed or open to the atmosphere, such as a zone of air indoor or outdoors) with a known spectrum of one or more molecules (in a computerized manner, using algorithms and/or machine learning), to determine whether the one or more molecules are present in the sample or not, possibly providing additional information such as a concentration or probability of presence of the one or more molecules in the sample.

One way of measuring the response spectrum is to perform experiments in which samples are controlled, and the associated spectrum is measured. This approach can be very costly, particularly in situations where one or more molecules in the samples are toxic or rare and can yield limited data sets. In principle, the quantum nature of the interactions which occur in quantum computers can be used to make this measurement based on quantum computations, which can be compared to simulating the evolution of the associated quantum system on the quantum computer. Indeed, the spectrum of one or more molecules following an electromagnetic interaction can be modelized as a response function of a quantum system which can evolve from an initial state to a final state along potentially different quantum paths, following an action of an effect, this response function can be computed using quantum computers, and quantum computers can be used to measure this response function based on an encoding of information in the quantum system. However, quantum computations on quantum computers are costly as well.

While existing techniques to determine the spectra of one or more molecules were satisfactory to a certain degree, there remained room for improvement. In particular, the computational costs of performing quantum computations on a quantum computer is tied to the efficiency of the quantum algorithms which are used, and there remains room for improvement in terms of devising more efficient quantum algorithms to reduce quantum computing costs.

### SUMMARY

Quantum systems essentially seek to control quantum interactions between different particles/fields and harness their quantum properties for human use. Quantum interactions typically involve the evolution of one or more quantum states over time. Examples of particles which can exhibit quantum behavior at the subatomic level include electrons and photons, but other approaches exist such as phonon-based approaches or cold atom/ion-based approaches. Various architectures exist for use as quantum systems and which are operable to control the evolution of quantum states, and the choice of a given architecture depends on the type of particles which are selected to exhibit quantum behavior in a given embodiment. Some architectures are more efficient for addressing certain types of problems. Quantum mechanics allows the quantum subsystems to be in a coherent superposition of more than one state simultaneously, a property which is fundamental to quantum computing.

In the case of many quantum systems, there is a particular challenge in predicting how one or more physical observables of the quantum system will react when perturbed in a particular way from an initial state. The field which addresses this challenge is known as quantum response theory and finds applications including spectroscopic properties (e.g., absorption, emission, Raman), circular dichroism effects, conductivity of nanowires, magnetic properties, electronic properties, chirality, etc.

Indeed, calculating response functions is exponentially hard when addressed classically via either time evolution or sums over excited states, especially in cases where there is a motivation to take into consideration some environmental dissipative and decoherence effects. In particular, the combinatorial explosion in the size of the Hilbert space can become particularly challenging for these systems, since response properties depend not only on a single ground state, but can also involve excited states. During a series of interactions, the system may simultaneously explore all excited states which are coupled by the interaction, an inherently quantum behaviour. To achieve suitable representativity, the modeled response may need to either access all the intermediary eigenstates of the Hamiltonian or the implementation of the time evolution under said Hamiltonian, tasks which are related through an uncertainty principle. Both obtaining eigenstates or performing time evolutions quickly can become prohibitively "expensive" as the size of the quantum system grows. Despite using multiple approximations and computational tricks, more complicated systems and effects often remain outside the existing computational capabilities of classical computers.

One way to tackle this issue involves a direct simulation of the time evolution of the quantum system on quantum computing hardware. This can be based on the principle that the time evolution of a given quantum Hamiltonian, also known as the Hamiltonian simulation problem, can be efficiently simulated on quantum hardware. Indeed, using product formula techniques, or linear combination of unitaries-based approaches, the cost of the Hamiltonian simulation on quantum hardware can be suitable.

Response theory can be considered as a perturbational theory, having that linear response covers the first-order reaction of the system to some interaction, whereas nonlinear response covers the higher orders. In particular, optical response theory has received significant attention due to its direct connection with spectroscopic experiments.

In the case of spectroscopy, in particular, a challenge can reside in determining the spectrum following a quantum electromagnetic interaction with one or more molecules (e.g. a single molecule, a group of molecules which do not interact with one another or a group of molecules which interact with one another). The type of electromagnetic interaction can depend on the type of spectroscopy technique being used. For instance in the case of infrared, mid-infrared, near-infrared spectroscopy (NIRS), and X-Ray absorption spectroscopy (XAS), the electromagnetic interaction is between the one or more molecules and photons. In the case of electron energy loss spectroscopy (EELS), the electromagnetic interaction is between the one or more molecules and electrons. Different spectroscopy techniques can be performed or modeled in different ways.

For several spectroscopy techniques, including NIRS, XAS and EELS, the spectrum can be modeled as a response function and the one or more molecules and electromagnetic interaction can be modeled as a quantum system and operators in accordance with the first-order dipole approximation. Using the first-order dipole approximation involves modeling each instance of electromagnetic interaction by applying a dipole operator on the quantum system. The quantum system and interaction can be encoded in a register of the quantum computer. More specifically, the one or more molecules can be modeled as a Hamiltonian, and the electromagnetic interaction in a target orientation can be modeled as a dipole operator, and both can be encoded as an initial state (e.g., a ground state or a thermal state) of a system register of the quantum computer. More specifically, the initial state can be defined and normalized using a classical computer and prepared in the system register of the quantum computer using various state preparation techniques. In this context, the dipole operator can constitute both an effect and an observable. The first order response of the system encoding the problem information can be measured using the quantum computer.

In accordance with one aspect, there is provided a method of determining or measuring a spectrum stemming from an electromagnetic interaction with one or more molecules, the method comprising: performing a plurality of iterations including preparing an initial state of a Hamiltonian acted upon by a dipole operator in a system register of a quantum computer, the Hamiltonian encoding the one or more molecules and the dipole operator encoding the electromagnetic interaction in a target orientation, encoding information pertaining to a distribution of possible values of a delay for a time evolution into one or more auxiliary qubits of the quantum computer, the time evolution being between the initial state and a final state, the final state corresponding to a time at which expectation values of the dipole operator in a final orientation are measured, and encoding information pertaining to a product of the distribution with one or more expectation values into the system register, including applying time-evolution operators to the system register, controlled on the one or more auxiliary qubits; performing one or more Fourier transforms of the product of the distribution with the one or more expectation values; determining the spectrum based on the one or more Fourier transforms.

In some embodiments, the method can further include representing the initial state as a linear combination of eigenstates of the set of eigenstates.

In some embodiments, the computing the leading-order error operator can include performing a Baker-Campbell-Hausdorf (BCH) expansion, and further include: determining eigenstates of interest having significant overlap with the initial state based on the linear combination of the eigenstates; computing a weighted average of individual maximum time steps associated to different ones of the eigenstates of interest; and determining the maximum time step based on weighted average.

In some embodiments, the method can further include identifying, from the linear combination eigenstates, eigenstates which are outside a window of frequencies of interest for the spectrum; and removing, from the linear combination of eigenstates, eigenstates which are outside the window of frequencies of interest for the spectrum.

**In** some embodiments, the removing can include building a projection operator and applying the projection operator onto the linear combination of eigenstates.

In some embodiments, the preparing the initial state of the Hamiltonian can be based on the linear combination of eigenstates subsequently to said removing.

In some embodiments, the method can further include determining a number of wavenumbers between eigenvalues of an approximate solution and eigenvalues of an exact solution, calculating a buffer based on the number of wavenumbers, and factoring in the buffer into the window of frequencies.

In some embodiments, the time evolution is between the initial state and a final state, the final state corresponding to a time at which expectation values of the dipole operator in a final orientation are measured, wherein the steps of performing a plurality of iterations and performing the one or more Fourier transform are repeated for more than one target orientation with the final orientation corresponding to the target orientation.

In some embodiments, the repeating is performed for three orientations of a coordinate system corresponding to on-diagonals of a 3 by 3 matrix.

In some embodiments, the dipole operator corresponds to linear absorption spectroscopy.

In some embodiments, the steps of performing a plurality of iterations and performing the one or more Fourier transform are further repeated for more than one combination of the final orientation and the target orientation, with the final orientation different from the target orientation.

In some embodiments, the further repeating is performed for three orientations of a coordinate system corresponding to off-diagonals of a 3 by 3 matrix.

In some embodiments, the dipole operator corresponds to angular resolved spectroscopy.

In some embodiments, the encoding the information pertaining to the product includes applying time-evolution operators of the form *e^{-iHδt}* to the system register, controlled on the one or more auxiliary qubits, where H is the Hamiltonian and *δt* is one or more of the possible values, wherein the time steps form corresponding portions of *δt*.

In some embodiments, for each iteration: the encoding information pertaining to the distribution includes encoding the distribution of all possible values of the delay as an initial superposition into more than one of the one or more auxiliary qubits forming an auxiliary register of the quantum computer; the encoding information pertaining to the product includes applying the time evolution on the system register, multiplexed on the auxiliary register; said performing the one of the one or more Fourier transform includes applying a quantum Fourier transform to the auxiliary register, subsequently to said applying the time-evolution; and a measurement is performed of the auxiliary register subsequently to said applying one of the one or more Fourier transform, including sampling a frequency of the spectrum; and the method further includes building one or more histograms based on the measurements acquired from each one of the plurality of iterations, and obtaining the response spectrum from the one or more histograms.

In some embodiments, each iteration further includes determining whether a measurement of the system register corresponds to the initial state, wherein said building the histogram includes integrating the measurement of the auxiliary register to the histogram contingent upon said measurement of the system register corresponding to the initial state.

In some embodiments, the building the histogram includes normalizing the histogram based on the measurements acquired in each one of the iterations.

In some embodiments, each iteration further includes applying block encoding of the dipole in the target direction, applying a Hermitian conjugate of a preparation circuit to the system register, and measuring the system register, between said applying the time evolution and applying the Fourier transform.

In some embodiments, each iteration further includes: isolating one of said possible values, including sampling the distribution; performing a Hadamard test on the isolated value including applying a first Hadamard gate to one of the auxiliary qubits forming an ancilla qubit, thereby preparing the ancilla qubit into a superposition, applying said time evolution controlled on the ancilla qubit, and performing a measurement yielding a single random variable encoding a component of the product associated to the isolated value; and further comprising building a time series of the single random variables obtained from the plurality of iterations, and said performing one or more Fourier transforms includes computing a Fourier transform classically on the time series.

In some embodiments, the method can further include repeating said performing the Hadamard test including applying a Hermitian conjugate of a phase gate on the ancilla qubit, applying a second Hadamard gate on the ancilla qubit and measuring the ancilla qubit.

In some embodiments, the method can further include preparing an additional register with a state, and following measuring the ancilla qubit, performing a SWAP test between the additional register and the system register controlled by the ancilla qubit, and modifying the single random variables based on the output of the SWAP test.

In accordance with another aspect, there is provided a non-transitory computer-readable storage medium having stored thereon instructions which, when executed upon by a computing device, cause the computing device to perform a method such as described above.

In accordance with another aspect, there is provided a system for measuring a spectrum stemming from an electromagnetic interaction with one or more molecules, the system comprising a processor and a memory, the memory having stored thereon instructions which, when executed upon by the processor, cause the system to: perform a plurality of iterations, each iteration including preparing an initial state of a Hamiltonian acted upon by a dipole operator in a system register of a quantum computer, the Hamiltonian encoding the one or more molecules and the dipole operator encoding the electromagnetic interaction in a target orientation, encoding information pertaining to a distribution of possible values of a delay for a time evolution into one or more auxiliary qubits of the quantum computer, and encoding information pertaining to a product of the distribution with one or more expectation values into the quantum computer; compute a leading-order error operator based on one or more diagonalizable fragments of the Hamiltonian and a product formula approximating the time evolution; compute a set of eigenstates representing a classical solution of the Hamiltonian; compute product formula expectation values of the leading-order error operator for at least some eigenstates of the set of eigenstates; compute a maximum time step associated with a target error using the product formula expectation values; perform one or more Fourier transforms of the product of the distribution with the one or more expectation values in a discretized manner, over one or more time steps, the one or more time steps being equal to or lesser than the maximum time step; and determine the spectrum based on the one or more Fourier transforms.

In some embodiments, when executed upon by the processor, the instructions further cause the system to represent the initial state as a linear combination of eigenstates of the set of eigenstates.

In some embodiments, said compute the leading-order error operator includes performing a Baker-Campbell-Hausdorf (BCH) expansion, wherein, when executed upon by the processor, the instructions further cause the system to: determine eigenstates of interest having significant overlap with the initial state based on the linear combination of the eigenstates; compute a weighted average of individual maximum time steps associated to different ones of the eigenstates of interest; and determine the maximum time step based on the weighted average.

In some embodiments, when executed upon by the processor, the instructions further cause the system to: identify, from the linear combination eigenstates, eigenstates which are outside a window of frequencies of interest for the spectrum; and remove, from the linear combination of eigenstates, the eigenstates which are outside the window of frequencies of interest for the spectrum.

In some embodiments, the time evolution is between the initial state and a final state, the final state corresponding to a time at which expectation values of the dipole operator in a final orientation are measured, wherein the steps of performing a plurality of iterations and performing the one or more Fourier transform are repeated for more than one target orientation with the final orientation corresponding to the target orientation, and wherein the dipole operator corresponds to linear absorption spectroscopy.

In some embodiments, the steps of performing a plurality of iterations and performing the one or more Fourier transform are further repeated for more than one combination of the final orientation and the target orientation, with the final orientation different from the target orientation, and wherein the dipole operator corresponds to angular resolved spectroscopy.

In some embodiments, said encoding the information pertaining to the product includes applying time-evolution operators of the form *e^{-iHδt}* to the system register, controlled on the one or more auxiliary qubits, where H is the Hamiltonian and *δt* is one or more of the possible values, wherein the time steps form corresponding portions of *δt*.

In some embodiments, for each iteration: said encoding information pertaining to the distribution includes encoding the distribution of all possible values of the delay as an initial superposition into more than one of the one or more auxiliary qubits forming an auxiliary register of the quantum computer; said encoding information pertaining to the product includes applying the time evolution on the system register, multiplexed on the auxiliary register; said performing the one of the one or more Fourier transform includes applying a quantum Fourier transform to the auxiliary register, subsequently to said applying the time-evolution; and a measurement is performed of the auxiliary register subsequently to said applying one of the one or more Fourier transform, including sampling a frequency of the spectrum; wherein, when executed upon by the processor, the instructions further cause the system to build one or more histograms based on the measurements acquired from each one of the plurality of iterations, wherein said determine the response spectrum includes obtaining the response spectrum from the one or more histograms.

In some embodiments, each iteration further includes determining whether a measurement of the system register corresponds to the initial state, wherein said building the histogram includes integrating the measurement of the auxiliary register to the histogram contingent upon said measurement of the system register corresponding to the initial state.

In some embodiments, the building the histogram includes normalizing the histogram based on the measurements acquired in each one of the iterations.

In some embodiments, each iteration further includes applying block encoding of the dipole in the target direction, applying a Hermitian conjugate of a preparation circuit to the system register, and measuring the system register, between said applying the time evolution and applying the Fourier transform.

In some embodiments, each iteration further includes: isolating one of said possible values, including sampling the distribution; performing a Hadamard test on the isolated value including applying a first Hadamard gate to one of the auxiliary qubits forming an ancilla qubit, thereby preparing the ancilla qubit into a superposition, applying said time evolution controlled on the ancilla qubit, and performing a measurement yielding a single random variable encoding a component of the product associated to the isolated value; and further comprising building a time series of the single random variables obtained from the plurality of iterations; wherein said performing one or more Fourier transforms includes computing a Fourier transform classically on the time series.

In some embodiments, when executed upon by the processor, the instructions further cause the system to repeat said performing the Hadamard test including applying a Hermitian conjugate of a phase gate on the ancilla qubit, applying a second Hadamard gate on the ancilla qubit and measuring the ancilla qubit.

In some embodiments, when executed upon by the processor, the instructions further cause the system to prepare an additional register with a state, following said measuring the ancilla qubit, perform a SWAP test between the additional register and the system register controlled by the ancilla qubit, and modify the single random variables based on the output of the SWAP test.

In accordance with another aspect, there is provided a system for measuring a spectrum stemming from an electromagnetic interaction with one or more molecules, the system comprising a processor and a memory, the memory having stored thereon instructions which, when executed upon by the processor, cause the system to: perform a plurality of iterations, each iteration including preparing an initial state of a Hamiltonian acted upon by a dipole operator in a system register of a quantum computer, the Hamiltonian encoding the one or more molecules and the dipole operator encoding the electromagnetic interaction in a target orientation, encoding information pertaining to a distribution of possible values of a delay for a time evolution into one or more auxiliary qubits of the quantum computer, and encoding information pertaining to a product of the distribution with one or more expectation values into an auxiliary register of the quantum computer; compute a leading-order error operator based on one or more diagonalizable fragments of the Hamiltonian and a product formula approximating the time evolution; compute a set of eigenstates representing a classical solution of the Hamiltonian; compute product formula expectation values of the leading-order error operator for at least some eigenstates of the set of eigenstates; compute a maximum time step associated with a target error using the product formula expectation values; applying a circuit on the auxiliary register to perform one or more Fourier transforms of the product of the distribution with the one or more expectation values in a discretized manner, over one or more time steps, the one or more time steps being equal to or lesser than the maximum time step and storing a result of the one or more Fourier transforms back into the auxiliary register; performing one or more measurements of the auxiliary register to acquire the result of the one or more Fourier transform; and determine the spectrum based on the result of the one or more Fourier transforms.

In some embodiments, when executed upon by the processor, the instructions further cause the system to represent the initial state as a linear combination of eigenstates of the set of eigenstates.

In some embodiments, said compute the leading-order error operator includes performing a Baker-Campbell-Hausdorf (BCH) expansion, wherein, when executed upon by the processor, the instructions further cause the system to: determine eigenstates of interest having significant overlap with the initial state based on the linear combination of the eigenstates; compute a weighted average of individual maximum time steps associated to different ones of the eigenstates of interest; and determine the maximum time step based on the weighted average.

In some embodiments, when executed upon by the processor, the instructions further cause the system to: identify, from the linear combination eigenstates, eigenstates which are outside a window of frequencies of interest for the spectrum; and remove, from the linear combination of eigenstates, the eigenstates which are outside the window of frequencies of interest for the spectrum.

In some embodiments, the time evolution is between the initial state and a final state, the final state corresponding to a time at which expectation values of the dipole operator in a final orientation are measured, wherein the steps of performing a plurality of iterations and performing the one or more Fourier transform are repeated for more than one target orientation with the final orientation corresponding to the target orientation, and wherein the dipole operator corresponds to linear absorption spectroscopy.

In some embodiments, the steps of performing a plurality of iterations and performing the one or more Fourier transform are further repeated for more than one combination of the final orientation and the target orientation, with the final orientation different from the target orientation, and wherein the dipole operator corresponds to angular resolved spectroscopy.

In some embodiments, said encoding the information pertaining to the product includes applying time-evolution operators of the form *e^{-iHδt}* to the system register, controlled on the one or more auxiliary qubits, where H is the Hamiltonian and *δt* is one or more of the possible values, wherein the time steps form corresponding portions of *δt*.

In some embodiments, for each iteration: said encoding information pertaining to the distribution includes encoding the distribution of all possible values of the delay as an initial superposition into more than one of the one or more auxiliary qubits forming an auxiliary register of the quantum computer; said encoding information pertaining to the product includes applying the time evolution on the system register, multiplexed on the auxiliary register; said performing the one of the one or more Fourier transform includes applying a quantum Fourier transform to the auxiliary register, subsequently to said applying the time-evolution; and a measurement is performed of the auxiliary register subsequently to said applying one of the one or more Fourier transform, including sampling a frequency of the spectrum; wherein, when executed upon by the processor, the instructions further cause the system to build one or more histograms based on the measurements acquired from each one of the plurality of iterations, wherein said determine the response spectrum includes obtaining the response spectrum from the one or more histograms.

In some embodiments, each iteration further includes determining whether a measurement of the system register corresponds to the initial state, wherein said building the histogram includes integrating the measurement of the auxiliary register to the histogram contingent upon said measurement of the system register corresponding to the initial state.

In some embodiments, said building the histogram includes normalizing the histogram based on the measurements acquired in each one of the iterations.

In some embodiments, each iteration further includes applying block encoding of the dipole in the target direction, applying a Hermitian conjugate of a preparation circuit to the system register, and measuring the system register, between said applying the time evolution and applying the Fourier transform.

In some embodiments, each iteration further includes: isolating one of said possible values, including sampling the distribution; performing a Hadamard test on the isolated value including applying a first Hadamard gate to one of the auxiliary qubits forming an ancilla qubit, thereby preparing the ancilla qubit into a superposition, applying said time evolution controlled on the ancilla qubit, and performing a measurement yielding a single random variable encoding a component of the product associated to the isolated value; and further comprising building a time series of the single random variables obtained from the plurality of iterations; wherein said performing one or more Fourier transforms includes computing a Fourier transform classically on the time series.

In some embodiments, when executed upon by the processor, the instructions further cause the system to repeat said performing the Hadamard test including applying a Hermitian conjugate of a phase gate on the ancilla qubit, applying a second Hadamard gate on the ancilla qubit and measuring the ancilla qubit.

In some embodiments, when executed upon by the processor, the instructions further cause the system to prepare an additional register with a state, following said measuring the ancilla qubit, perform a SWAP test between the additional register and the system register controlled by the ancilla qubit, and modify the single random variables based on the output of the SWAP test.

In accordance with another aspect, there is provided a method of measuring a spectrum stemming from an electromagnetic interaction with one or more molecules, the method comprising: performing a plurality of iterations, each iteration including preparing an initial state of a Hamiltonian acted upon by a dipole operator in a system register of a quantum computer, the Hamiltonian encoding the one or more molecules and the dipole operator encoding the electromagnetic interaction in a target orientation, encoding information pertaining to a distribution of possible values of a delay for a time evolution into one or more auxiliary qubits of the quantum computer, and encoding information pertaining to a product of the distribution with one or more expectation values into an auxiliary register of the quantum computer; computing a leading-order error operator based on one or more diagonalizable fragments of the Hamiltonian and a product formula approximating the time evolution; computing a set of eigenstates representing a classical solution of the Hamiltonian; computing product formula expectation values of the leading-order error operator for at least some eigenstates of the set of eigenstates; computing a maximum time step associated with a target error using the product formula expectation values; applying a circuit on the auxiliary register to perform one or more Fourier transforms of the product of the distribution with the one or more expectation values in a discretized manner, over one or more time steps, the one or more time steps being equal to or lesser than the maximum time step and storing a result of the one or more Fourier transforms back into the auxiliary register; performing one or more measurements of the auxiliary register to acquire the result of the one or more Fourier transform; and determining the spectrum based on the acquired result of the one or more Fourier transforms.

In some embodiments, for each iteration: said encoding information pertaining to the distribution includes encoding the distribution of all possible values of the delay as an initial superposition into more than one of the one or more auxiliary qubits forming an auxiliary register of the quantum computer; said encoding information pertaining to the product includes applying the time evolution on the system register, multiplexed on the auxiliary register; said performing the one of the one or more Fourier transform includes applying a quantum Fourier transform to the auxiliary register, subsequently to said applying the time-evolution; and a measurement is performed of the auxiliary register subsequently to said applying one of the one or more Fourier transform, including sampling a frequency of the spectrum; further comprising building one or more histograms based on the measurements acquired from each one of the plurality of iterations, wherein said determining the response spectrum includes obtaining the response spectrum from the one or more histograms.

In some embodiments, each iteration further includes determining whether a measurement of the system register corresponds to the initial state, wherein said building the histogram includes integrating the measurement of the auxiliary register to the histogram contingent upon said measurement of the system register corresponding to the initial state.

In some embodiments, each iteration further includes: isolating one of said possible values, including sampling the distribution; performing a Hadamard test on the isolated value including applying a first Hadamard gate to one of the auxiliary qubits forming an ancilla qubit, thereby preparing the ancilla qubit into a superposition, applying said time evolution controlled on the ancilla qubit, and performing a measurement yielding a single random variable encoding a component of the product associated to the isolated value; and further comprising building a time series of the single random variables obtained from the plurality of iterations; wherein said performing one or more Fourier transforms includes computing a Fourier transform classically on the time series.

In some embodiments, the method further comprises repeating said performing the Hadamard test including applying a Hermitian conjugate of a phase gate on the ancilla qubit, applying a second Hadamard gate on the ancilla qubit, measuring the ancilla qubit preparing an additional register with a state, following said measuring the ancilla qubit, performing a SWAP test between the additional register and the system register controlled by the ancilla qubit, and modifying the single random variables based on the output of the SWAP test.

In accordance with another aspect, there is provided a non-transitory computer-readable storage medium having stored thereon instructions which, when executed upon by a computing device, cause the computing device to perform a method comprising: performing a plurality of iterations, each iteration including preparing an initial state of a Hamiltonian acted upon by a dipole operator in a system register of a quantum computer, the Hamiltonian encoding one or more molecules and the dipole operator encoding an electromagnetic interaction with the one or more molecules in a target orientation, encoding information pertaining to a distribution of possible values of a delay for a time evolution into one or more auxiliary qubits of the quantum computer, and encoding information pertaining to a product of the distribution with one or more expectation values into the quantum computer; computing a leading-order error operator based on one or more diagonalizable fragments of the Hamiltonian and a product formula approximating the time evolution; computing a set of eigenstates representing a classical solution of the Hamiltonian; computing product formula expectation values of the leading-order error operator for at least some eigenstates of the set of eigenstates; computing a maximum time step associated with a target error using the product formula expectation values; performing one or more Fourier transforms of the product of the distribution with the one or more expectation values in a discretized manner, over one or more time steps, the one or more time steps being equal to or lesser than the maximum time step; and determining a spectrum based on the one or more Fourier transforms.

The time-evolution operators can be of the form *e^{-iHδt}*, where *H* is the Hamiltonian and *δt* is one or more of the possible values. It will be noted that in this specification, a "register" refers to a group of qubits dedicated to a given purpose in the context of a quantum computation.

While such a method can be satisfactory to a certain degree, there can remain room for improvement in some embodiments. Indeed, reducing computational costs, while achieving a suitable degree of accuracy, can remain a constant concern.

In accordance with one aspect, in practice, the Fourier transform cannot be performed as a whole and needs to be discretized. More specifically, each one of the one or more Fourier transforms may need to be evaluated for different time steps, the different time steps constituting different portions of *δt*.

Indeed, when discretizing the Fourier transform, a grid of delays becomes associated to it. The grid has 2 attributes: width and number of points. The width is related to the spectral window in which the spectrum is to be resolved. The number of points is related to accuracy (for given window width). The *δt* is fixed by the spectrum/spectral window.

Discretizing the Fourier transform, whether done when implementing the time evolution in the time domain or in the frequency domain, imparts some error. The amplitude of the error is related to the size of the step in accordance with a relationship for which the details depend on the problem, but which typically involves increasing the error with increasing step size. On the other hand, increasing step size typically reduces the number of iterations, and thus computing costs. There thus remained a need for a way of determining a step size which is as large as possible, while remaining below a certain error threshold which may be predetermined. In principle, such a calculation of maximum step size can allow setting the step size to that maximum step size value, or slightly below it, in a manner to ensure a target accuracy while minimizing the number of iterations and computing costs.

It was found possible to obtain a suitable determination of such a maximum step size value using a computerized process. More specifically a leading-order error operator can be computed based on fragments of the Hamiltonian which can be diagonalized, and a product formula approximating the time evolution. This can be achieved using a Baker-Campbell-Hausdorf (BCH) expansion, for instance. At a same or different time, a set of eigenstates representing a classical solution of the Hamiltonian can be computed. Then, product formula expectation values of the leading-order error operator can be computed for some or all of the eigenstates of the set. A maximum time step can then be determined for a target error (error threshold) using the product formula expectation values, and the method of determining or measuring the spectrum can be performed with the time step set at or below the maximum time step.

In practice, for many Hamiltonians for which it is relevant to use a quantum computer, the number of eigenstates increases exponentially, and there can be a motivation to perform the process for only some of the of the eigenstates of the set. One way of selecting only some of the eigenstates is to first compute the initial state as a linear combination of the eigenstates, and then identify, amongst the eigenstates, some having a significant overlap with the initial state. Then, a weighted average of individual maximum time steps associated to different ones of the eigenstates identified can be computed, and the maximum time step can be based on the weighted average, as opposed to, for example, selecting the maximum time step based on the minimum value of the maximum time steps calculated for all of the eigenstates.

Accordingly, in accordance with a first aspect, there is provided a method of determining or measuring a spectrum stemming from an electromagnetic interaction with one or more molecules, the method comprising: performing a plurality of iterations including preparing an initial state of a Hamiltonian acted upon by a dipole operator in a system register of a quantum computer, the Hamiltonian encoding the one or more molecules and the dipole operator encoding the electromagnetic interaction in a target orientation, encoding information pertaining to a distribution of possible values of a delay for a time evolution into one or more auxiliary qubits of the quantum computer, and encoding information pertaining to a product of the distribution with one or more expectation values into the quantum computer; computing a leading-order error operator based on fragments of the Hamiltonian which can be diagonalized and a product formula approximating the time evolution; computing a set of eigenstates representing a classical solution of the Hamiltonian; computing product formula expectation values of the leading-order error operator for at least some eigenstates of the set; computing a maximum time step associated to a target error using the product formula expectation values; performing one or more Fourier transforms of the product of the distribution with the one or more expectation values in a discretized manner, over one or more time steps, the one or more time steps being equal to or lesser than the maximum time step; and determining the spectrum based on the one or more Fourier transforms.

In accordance with another aspect, there is provided a method of determining a spectrum for one or more molecules, the method including computing a leading-order error operator based on fragments of a Hamiltonian representing the one more molecules, where the fragments can be diagonalized, and a product formula approximating the time evolution; computing a set of eigenstates representing a classical solution of the Hamiltonian; computing product formula expectation values of the leading-order error operator for at least some eigenstates of the set; computing a maximum time step associated to a target error using the product formula expectation values; and performing a quantum phase estimation quantum computing process to determine the spectrum, including performing one or more Fourier transforms of the product of the distribution with the one or more expectation values in a discretized manner, over one or more time steps, the one or more time steps being equal to or lesser than the maximum time step.

In accordance with one aspect, there is provided a method of measuring a spectra using a quantum computer. The spectra contain the information about how an observable of the quantum system evolves from an initial state to a final state due to the electromagnetic interaction. In practice, the measurement will have a certain level of precision and will involve an iterative process having a certain number of repetitions of a sequence of several steps. In one embodiment, referred to hereinbelow as the "frequency-domain implementation", the measurement may yield a histogram with amplitudes varying as a function of frequency, which can be a suitable discrete approximation of a continuous response function, and the degree of precision with which the histogram corresponds to the response function can be controlled by controlling the number of repetitions of the iterative process. In another embodiment, referred to hereinbelow as the "time-domain implementation", the measurement may directly obtain the approximation of the response function via a Fourier transform of a time series built using an iterative process, rather than proceeding via the construction of a histogram. The response function may be used, for instance, to retrieve the spectrum. The dipole operator and the Hamiltonian can be expressed in the form of a sum of Hermitian terms or unitaries in a classical computer.

More specifically, in the frequency-domain implementation, a number of registers may be used. These registers can be referred to herein as auxiliary registers. The application of the operators can be multiplexed, i.e., be controlled on all of the auxiliary registers which encode the corresponding delay, on all the possible values encoded in the registers. In the time-domain implementation, the correlation function circuit can be applied in the context of a Hadamard test, and the application of the operators can be controlled on an ancilla qubit. For instance, in the case of the time-domain method, a specific value of a delay is determined via a Monte Carlo method. In the frequency-domain method, various possible values of the delay are implemented in parallel through multiplexing.

Building the histogram can include building the histogram based on the binary encoding of frequencies acquired at each "successful" iteration, while not integrating the binary encodings of frequencies acquired from the "unsuccessful" iterations. In some embodiments, the measurements made during the "unsuccessful" iterations may nonetheless be used to contribute to a normalization factor of the histogram, together with the measurements made during the "successful" iterations.

At this stage, the histogram may be representative specifically of the contribution of a single target orientation to the response function, which may be considered a suitable measurement of the response function in some cases.

In embodiments where multiple target orientations are to be considered, the process can be repeated for each one of the target orientations which are to be considered, until all the significant contributions have been accounted for.

In one example, the additional contributions can be accounted for by summing the histograms corresponding to all the different orientations, and the response function may be obtained from the sum of the histograms. In some alternate examples, the latter approach would not be suitable and additional steps may need to be taken. Indeed, the histogram represents a squared amplitude. While the squared amplitude may provide all the information needed, it may not represent negative contributions which may be significant in some cases. If the response is not real, but complex, it may be relevant to separate the real and imaginary contributions.

When one wants to measure an expectation value of the observable, the expectation value can be extracted from the response function. Every time the frequency circuit is run, one or more frequencies can be sampled from this distribution. By running the circuit many times, the distribution can be rebuilt to arbitrary accuracy.

The next section describes an alternative way of measuring the response function which will be referred to as a "time-domain implementation".

Rather than using a process such as presented above, which will be referred to herein as being in the frequency-domain implementation, a process which will be referred to herein as being in the time-domain implementation can alternately be used.

A general concept behind the time-domain implementation is to implement a particular value of a delay (by contrast with a number of possible values of the delay applied in parallel), obtain the single random variable, and repeat for many values of the delay to build the time series of the single random variable. The Fourier transform can then be done classically by taking the sum over delays with exp(-*iωt*) for each different delay.

It will also be noted that by contrast with the frequency-domain implementation, in the time-domain implementation, the time evolutions are not multiplexed (applied over all different values). Rather, a single value is obtained using the Monte Carlo process, and the time evolutions are then implemented for that specific value. The Monte Carlo process can repeat for a given number of times, until a desired level of statistical representativity is met.

The Hadamard test can be repeated many times to obtain a target level of statistical representativity.

In the context of this specification, both the frequency domain and the time domain approach will be referred to as quantum phase estimation (QPE) processes.

In this specification, all technical implementation details and advantages described with respect to a particular aspect are self-evidently mutatis mutandis applicable for all other aspects.

Many further features and combinations thereof concerning the present improvements will appear to those skilled in the art following a reading of the instant disclosure.

### DESCRIPTION OF THE FIGURES

In the figures,
Fig. 1 is a schematic view of an example quantum computer;
Fig. 2 is a flow chart representing embodiments of a method for determining a spectrum of an electromagnetic interaction with one or more molecules with a quantum computer;
Fig. 3 is an example circuit which can be used to compute an expectation value associated with cross (off-diagonal) terms and diagonal terms;
Fig. 4A and 4B are example flow charts representing embodiments of the method of Fig. 2, implemented in the frequency domain and in the time domain, respectively;
Fig. 5 is a schematic diagram representing workflow description for obtaining an NIRS spectrum on a quantum computer, where titles in bold inside sub-blocks constitute tasks, with underlying text describing an associated technique;
Fig. 6 is a graph presenting a real-space representation of single vibrational mode q, where each of the 2^{N} integers associated with the N-qubit computational basis states are associated with a different point in the grid discretizing q, the wavefunction |ϕ〉 then being represented by a coherent superposition over computational basis states with associated coefficients ϕ(q⁽ⁿ⁾), namely |ϕ〉 = Σₙ ϕ(q⁽ⁿ⁾)|q⁽ⁿ⁾〉;
Fig. 7 is a graph showing simulated spectrum of hydrogen sulfide (H₂S) molecule, where the amplitudes of the near-IR spectrum are significantly smaller than those of the mid-IR and the former come from overtones and combination bands, having a strong anharmonic character that is classically challenging to simulate;
Fig. 8 is a Hadamard test circuit for a given evolution time t whereby obtaining expectation values over a range of times and performing the Fourier transform on classical hardware;
Fig. 9 is a double measurement Hadamard circuit which performs a SWAP test on the output of the Hadamard circuit, returning additional information about the expectation value 〈*ψ_{ρ}*|*Û*|*ψ_{ρ}*〉;
Fig. 10 is a double phase circuit which effectively doubles the time-evolution length on the Hadamard test in Fig. 8;
Fig. 11 is a quantum arithmetic-based circuit to implement the evolution associated with an exponential *e*^{-*i*Φ*^{ij}q̂ⁱq̂ⁱ*} where the Mult operation corresponds to an out-of-place signed multiplier and Add to the addition circuit for the phase gradient technique and the *̅X̅*̅(Φ*ᵢⱼ*) operation is purely composed of Pauli X gates, directly loading the binary representation of Φ*ᵢⱼ* on the quantum register using *bₖ* bits of precision; here the size of the registers carrying the multiplication results increases as a function of the degree of the carried monomial term;
Fig. 12 are graphs simulating NIRS spectra for hydrogen sulfide H₂S (top) and formaldehyde CH₂O (bottom) molecules using three qubits per vibrational coordinate and a fourth-degree 2-mode Taylor expanded Hamiltonian for both, here shown r's were obtained using the perturbative approach for both *ε_{ν}* = 1*cm*⁻¹ and *ε_{ν}* = 10*cm*⁻¹, exact expectation values were considered when reconstructing the spectra, which amounts to taking an infinite amount of samples from the Hadamard tests and only the NIRS region has been plotted;
Fig. 13 are graphs of reconstructed randomly generated NIRS spectrum with support Ω = 9,000 *cm*⁻¹ and Lorentzian widths *η =* 10 *cm*⁻¹, where explored hyperparameters consist of the Fourier integral accuracy *ε* (top) and number of shots 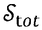 (bottom);

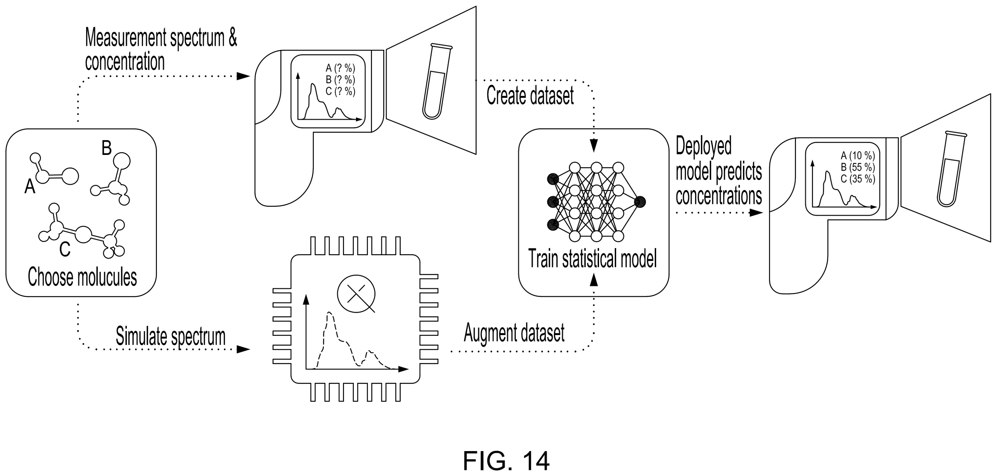

Fig. 14 is a schematic view of an example embodiment of chemical detection using first order dipole spectroscopy such as NIRS where, once a molecule is chosen, a statistical model is trained that can then infer the concentration of the molecule by detecting a signature of its spectrum in an unknown sample, and where the training dataset and training process of the statistical model can be augmented using simulated spectra obtained on a quantum computer;
Fig. 15 shows another example of a quantum computer; and
Fig. 16 shows an example of a classical computer.

### DETAILED DESCRIPTION

Various technologies of hosting quantum information have been developed over the years, and many more technologies are expected to continue to surface. Some technologies use electrons, for instance, wherein the state of the spin can be used to encode quantum information (e.g., spin up vs. spin down). Other technologies use photons, where the state of the polarization can be used to encode quantum information (e.g., vertical polarization vs. horizontal polarization), or bosonic encoding may be used. The encoding scheme can be binary (e.g., spin up vs. spin down) or continuous variable based. In one example of an architecture using bosonic encoding, a plurality of Gaussian Boson Sampling (GBS) sources each configured to generate a continuous-variable (CV) state such as a Gottesman-Kitaev-Preskill (GKP) state may be used, and data may be mapped onto the generated CV states. Such an embodiment can be implemented in a photonic architecture where the CV states are states of light, for instance.

Various architectures of quantum computers have been developed in recent years, such as architectures based on superconducting circuits, quantum dots, trapped ions, photonic circuits, phonons, cold atoms, and hybrid approaches. Some architectures are perceived as more promising than others, but the overall maturity of the field is such that breakthrough, game-changing innovations are expected to continue to occur. It will be understood that while existing hardware and process steps have led to the relatively intense level of international excitement about the possibilities opened up via quantum computing, there remains much room for improvement, and many further developments will be required before quantum computers can be offered in the form of commercially available products.

In the case of many architectures, the quantum behavior is exhibited in a cryogenic environment, such as below 100 K, 50 K, 10 K or even lower, depending on the architecture, although research for quantum systems operable at higher temperatures is a highly active area of current research. Such temperatures can be sustained with a specialized refrigerator referred to as a cryostat. One feature of some photonic architectures is that the quantum states may be exhibited in a non-cryogenic environment (e.g., photonic modes used as qubits "living" at room temperature). However, even in photonic architectures, it is common to have some elements of the system operate in a cryostat, such as may be the case with some elements of a measurement system (e.g., photon number resolving (PNR) detectors).

The basic concept of performing a computation in a quantum context typically involves a quantum interaction such as entanglement between quantum states of one or more particles referred to as quantum subsystems. A typical approach to quantum computing is to make definitions of qubits based on possible quantum states in the quantum subsystem. This latter process is commonly referred to as encoding. Indeed, in a process of quantum computation, information can be encoded in the form of eigenstates of quantum subsystems. Particular hardware and complex control schemes, typically encoded as software functions and executed by a classical computer, are typically required. The definition of the qubits is typically stored in a memory of the classical computer. Different types of hardware which can host various types of quantum states exist, and the selection thereof depends on the nature of the quantum subsystem used as a resource in the quantum computation.

Different forms of quantum computing exist, which typically involve using two or more quantum subsystems interconnected to one another in a manner to allow quantum interaction, typically quantum entanglement.

In adiabatic quantum computing, a specific problem is posed in the form of a configuration of interconnection between quantum subsystems which can communicate directly with one another, and the solution to the problem appears in the form of a ground state of the overall system.

In gate-based quantum computing, which can provide a "universal" computing approach, quantum gates are applied in a controlled manner. There are two common types of approaches. In the standard circuit-based model of quantum computing, quantum states are initialized in the form of a plurality of quantum subsystems, and a sequence of gates referred to as a circuit is applied by controlling the quantum interaction between some of the quantum subsystems using devices commonly referred to as couplers. The couplers are used to selectively allow or prevent the interaction between corresponding quantum subsystems. A measurement is then applied to the quantum subsystems to conclude the quantum computation.

Measurement-based quantum computation (MBQC) differs from standard circuit-based quantum computation but has been shown to have the potential to be mathematically equivalent. In measurement-based quantum computation, quantum states are initialized and entangled into cluster states. The application of the gates is performed by the way the measurement is applied, and more specifically, as a sequence of adaptive measurements where the way subsequent measurements are performed depends on the results of previous measurements. In MBQC, measurements of the quantum states (e.g., homodyne measurements of entangled photonic states) are what actually perform the quantum logic operations. In one example of photonic MBQC, a number of photonic states (e.g., GKP states, squeezed states) are produced and then entangled into a "cluster state," (e.g., a 3D lattice of optical modes). Measurements are then performed on the cluster state. These measurements are used for error correction and to perform MBQC. Therefore, in MBQC, quantum "gates" are implemented by the measurements, though MBQC maps perhaps less directly to the "circuits" of the standard circuit-based model.

Other forms of quantum computing applications can include quantum communication routers, for instance. The exact nature of the quantum subsystems and of any couplers will vary depending on the type of quantum architectures in which they are implemented.

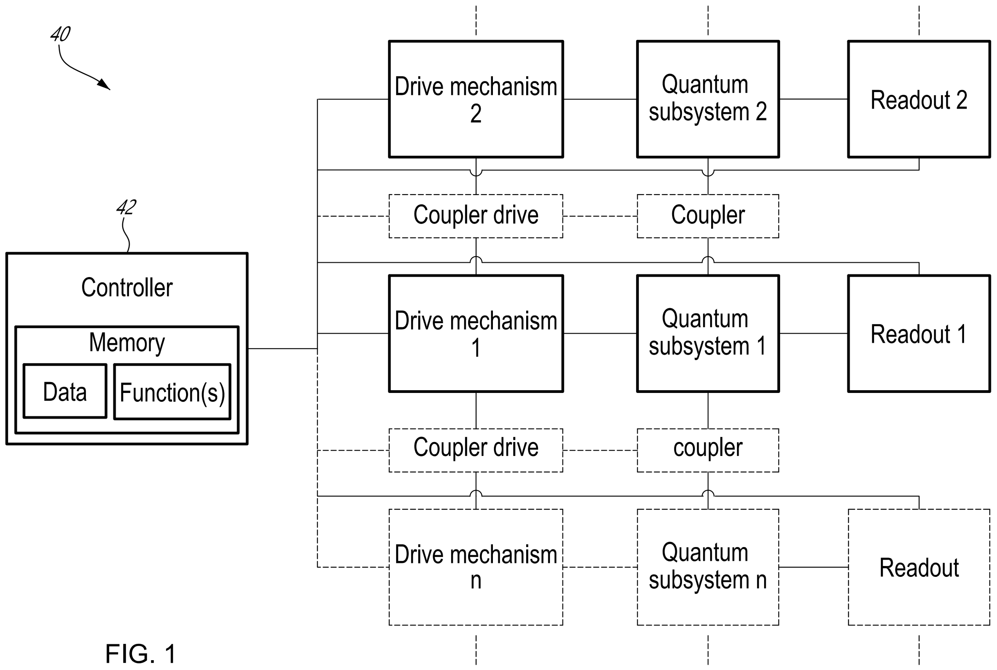

Fig. 1 presents a highly simplistic example of a quantum computer operating on the basis of circuit-based quantum computations. The quantum computer shown in Fig. 1 has a quantum system which includes a plurality of quantum subsystems. In the context of quantum computing, the quantum subsystems are used to host logical states and can be referred to as qubits, and the quantum system can be referred to as a quantum processor 40. Typical quantum computers have many quantum subsystems, such as 10's of qubits, or more. **In** the context of performing a quantum computation, groups of more than one qubit can be reserved for certain specific functions, and referred to as registers. The nature of the qubits, the means by which logical states are hosted in the qubits, and the means by which operations are performed between the qubits depend on the choice of architecture.

In many applications where qubits are based on bosonic encoding, for example, the logical states may be driven in some form of resonator using some form of driving hardware. The driving hardware is controlled by a component which will be referred to herein as a controller for simplicity, and which typically includes a classical computer (i.e., a computer which uses binary bits rather than qubits to encode information). The qubits are often refrigerated to very low temperatures and insulated from the external environment. In an adiabatic quantum computing architecture, the quantum subsystems can be directly operably interconnected to one another. In circuit-based quantum computing, the quantum subsystems are typically interconnected to one another via couplers which are used to selectively control the interactions between the quantum subsystems. The couplers can also be quantum subsystems operable to host states via which the logical states of the two or more connecting quantum subsystems are to interact, and are also driven by driving hardware which can be controlled by the same controller for convenience. Readout ports can be provided via which the state of the qubits can be read, an operation which can be said to involve "measuring."

The controller 42 can be expected to include a memory which can include computer-readable instructions executable to perform functions driving the operation of the controller 42 and devices it controls, and data. The functions can include a "driving program," for instance, which, in the case of circuit-based quantum computing, can include a sequence of gates, typically referred to as a quantum circuit, stored as data in the memory. When the states of the qubits are read, the measured values can be stored in the form of data, for instance.

Performing a quantum computation can involve a quantum interaction, such as entanglement, between the physical states (which encode information) of the different quantum subsystems. However, for a quantum computation to occur, one may further actively initialize the physical states of the quantum subsystems prior to the quantum interaction (e.g., control the physical states of the quantum subsystems), and actively measure the physical states subsequently to the quantum interaction. Moreover, when performing a quantum computation in a circuit-based model, one may additionally actively control the interaction via a coupler which is changed from a configuration where it impedes interaction to a configuration where it favors interaction, and then back.

Henceforth, performing a given quantum computation can involve the steps of initializing, interacting, and measuring. "Interacting" can involve applying one or more gates or encoding, and can be performed in accordance with a quantum circuit, typically referred to as a "circuit" for short. Accordingly, any of the steps of preparing, initializing, interacting, and measuring may have corresponding hardware elements and an active control process associated therewith.

Any active control step may require associated elements of hardware. Moreover, in practical implementations, it is impossible to manually control the hardware associated with the active control steps in a context of the amount of time available to perform any one of these steps. Accordingly, automated control can be used to implement the active control steps. The automated control processes can take the form of associated functions defined by corresponding software modules. Accordingly, several elements of hardware, such as preparing hardware, initializing hardware, coupler control hardware, and measuring hardware may involve automated, active control in the context of performing a single quantum computation. The control functions for such hardware elements may be performed via corresponding software modules which can be in the form of computer-readable instructions driving the operation of the controller.

The components of the quantum processor, such as the quantum subsystems and some or all of the control hardware, are typically refrigerated to very low temperatures and insulated from the environment and can therefore be said to be inside a "refrigerator," which can be a dilution refrigerator for instance. Components of the classical computer such as its processor and its non-transitory memory can be located outside the refrigerator. Electrical connections can therefore extend across an enclosure of the refrigerator, between the ambient temperature environment and the cryogenic environment. The combination of the classical computer, of the quantum processor, of the electrical connections, and of the refrigerator can be referred to collectively as a "quantum computer."

While a simplistic scenario having a minimum of 2 quantum subsystems is schematized in solid lines in Fig. 1, in practice, it can be preferred to embody quantum computers with a significantly greater number of quantum subsystems, or qubits, which can significantly affect the complexity of the overall system, both from the hardware and from the control process points of view.

For several spectroscopy techniques, including NIRS, XAS and EELS, the spectrum can be modeled as a response function, and the one or more molecules and electromagnetic interaction can be modeled as a quantum system and operators in accordance with the first-order dipole approximation.

First-order dipole approximation refers to scenario where absorption cross-section *σ_{A}*(*ω*), a function indicating the strength of absorption at a particular frequency of incoming radiation *ω* can be considered to be given by ${σ}_{A} \left(ω\right) ∝ ω {\sum }_{ρ = x , y , z} {\sum }_{f} {\left|\left〈{E}_{f}\left|{\hat{μ}}_{ρ}\right|{E}_{0}\right〉\right|}^{2} {L}_{η} \left(ω-{E}_{f}+{E}_{0}\right) .$ (1)

Here |*E*₀〉, |*E_{f}*〉 are an initial (e.g., ground) and final (e.g., excited) states of a molecular vibrational Hamiltonian *Ĥ*, *ω* is the frequency of incoming radiation, *µ̂_{ρ}* is the *ρ*-th Cartesian component of the molecular dipole operator, and 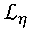 (*ω*) *= η*/(*ω*² + *η*²) is the Lorentzian broadening with *η* its half-width at half-maximum. This broadening is related to the lifetime of excited states of the system as well as the resolution of the measurement apparatus, and in practice usually takes values between *η* = 1 cm ⁻¹ and 10 cm ⁻¹.

In the specific case of NIRS, the set of peak positions *E_{f}* and absorption intensities |〈*E_{f}*|*µ̂_{ρ}*|*E*₀〉|² is unique to a given molecule, being determined by the details of its ground-state potential energy surface (PES). Since peaks and their intensities can determine the absorption spectrum, that implies the spectrum itself can be a unique *fingerprint* of the molecule. Thus, if molecular spectra are cataloged *a priori,* the presence of a given molecule in an unknown sample can be determined through a direct comparison against the catalog. In other first-order dipole spectroscopy techniques, such as XAS and EELS, this fingerprint may further incorporate information from the excited electronic states as well.

More specifically, using the first-order dipole approximation involves modeling each instance of electromagnetic interaction by applying a dipole operator on the quantum system. The quantum system and interaction can be encoded in a register of the quantum computer. More specifically, the one or more molecules can be modeled as a Hamiltonian, and the electromagnetic interaction in a target orientation can be modeled as a dipole operator, and both can be encoded as an initial state (e.g., a ground state or a thermal state) of a system register of the quantum computer. More specifically, the initial state can be defined and normalized using a classical computer and prepared in the system register of the quantum computer using various state preparation techniques. In this context, the dipole operator can constitute both an effect and an observable. The first order response of the system encoding the problem information can be measured using the quantum computer. In the context of the first-order, the dipole operator acts a single time on the Hamiltonian.

More specifically, the spectrum (FT1) is in the frequency domain, and is known to correspond to a convolution of a correlation function (FT2) with a lineshape (FT3 - which is known and a function of the problem), both also functions in the frequency domain. In other words, $FT 1 = FT 2 * FT 3 .$

Using the convolution theorem, the spectrum is thus also the result of performing a Fourier Transform on the product of the time domain functions ft2 and ft3, each representing an inverse Fourier Transform of FT2 and FT3, respectively. ft2 can be referred to as "expectation values" herein, whereas ft3 can be referred to as "distribution over time". In other words: $FT 1 = Fourier Transform of \left(ft2⋅ft3\right) .$

A quantum computer can be helpful in performing this operation which, in practice, involves breaking down the integral associated to the Fourier Transform into a sum, processing components of the sum in the quantum computer and then reconstructing. The breaking down can be done either in the time domain or the frequency domain. In the frequency domain approach, a distribution of possible values for a delay of the electromagnetic interaction in accordance with the lineshape can be encoded at the same time, and an individual frequency of the spectrum can directly be sampled with the quantum computer, including performing the Fourier transform in the quantum computer. Various iterations of the process lead to sampling different frequencies of the spectrum, from which the full spectrum can be reconstructed. In the time domain approach, the distribution can be sampled (e.g., using a typical Monte Carlo process or, equivalently, shot allocation) to isolate a value, and Hadamard test can be performed with the quantum computer at the isolated value, and outputting a single random variable. Repeating several different iterations of the process, for different ones of the sampled values and repeating multiple times for same ones of the sampled values, leads to outputting a time series of single random variables, from which the spectrum can be recovered by classically performing a Fourier transform. In both cases, the process performed on the quantum computer can involve encoding information pertaining to a product of the distribution with the one or more expectation values performing controlled time evolution. In some embodiments, there can be quantum advantage to performing the operations on a computer instead of performing the operations on a classical computer.

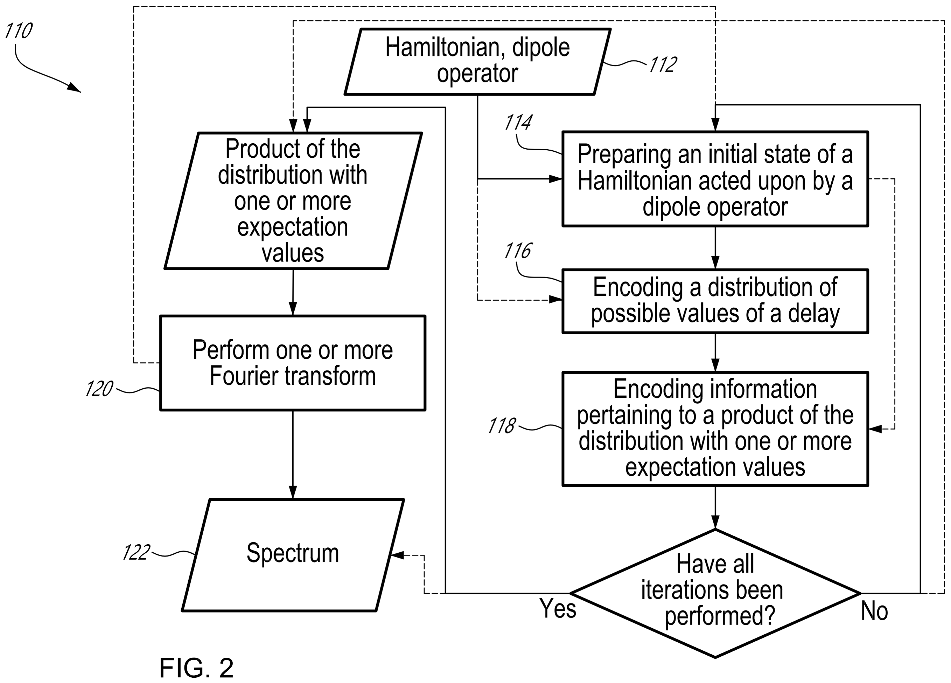

In some embodiments, a first-order response spectrum stemming from an electromagnetic interaction with one or more molecules, in accordance with the "dipole" approximation, can be determined or measured 110 as shown in Fig. 2. Firstly, a Hamiltonian and a dipole operator pertaining to the specifics of the problem at hand can be obtained 112 (e.g., provided as inputs). More specifically, the Hamiltonian can encode the one or more molecules, and the dipole operator can encode the electromagnetic interaction in a target orientation. A delay for a time evolution can be associated to the Fourier transform, and a distribution of possible values of the delay can be known and associated to the problem. Several iterations can be performed on the quantum computer. Each iteration can include, on the one hand, the preparation 114 of an initial state of the Hamiltonian acted upon by the dipole operator in the target orientation in a system register of the quantum computer. Each iteration can include, on the other hand, encoding 116 information pertaining to the distribution of possible values of a delay for a time evolution into one or more auxiliary qubits of the quantum computer. The delay can be between the initial state and a final state, and the final state can correspond to a time at which expectation values of the dipole operator in a final orientation are measured. The final orientation can be the same as the initial orientation, or different than the initial orientation. Then, the time evolution is applied on the quantum computer, simulating the time evolution between the initial state and the final state, thereby encoding 118 information pertaining to a product of the distribution with one or more expectation values into the system register. This can involve applying time-evolution operators of the form *e^{-iHδt}* to the system register, controlled on the one or more auxiliary qubits, where H is the Hamiltonian and *δt* is one or more of the possible values. One or more Fourier transforms of the product of the distribution with the one or more expectation values are performed 120, and the spectrum 122 is determined based on the one or more Fourier transforms. In Fig. 2, some potential alternate paths which can be taken are presented in dashed lines.

There are two main families of implementation of a method such as presented above, which will be referred to herein as the frequency domain approach and the time domain approach. While some elements can be common to both approaches, there are several differences. More specifically, the delay is related to the Fourier transform. The Fourier transform is performed over the delay, and the Fourier transform is discretized into time steps. Indeed, in the frequency domain approach, the step of encoding a distribution of possible values of the delay is encoded simultaneously as an initial superposition in a plurality of auxiliary qubits forming an auxiliary register, the time evolution is applied in a multiplexed manner with the auxiliary register, and the Fourier transform is performed on the quantum computer, sampling for one frequency per iteration. Histograms are built based on the measurements acquired, and the spectrum is reconstituted from the histograms. In the time domain approach, the step of encoding a distribution of possible values of the delay involves sampling the distribution to isolate one of the values, and performing Hadamard tests using the isolated value, with the different iterations performed on different values, leading to a time series of single random variables, and the Fourier transform is performed classically on the time series. While the route taken can be very different in the time domain vs. the frequency domain approaches, a similar result can be achieved.

More specifically, in either case, one may define a problem specification, picking one or more molecule, a type of spectroscopy, obtaining an associated Hamiltonian, etc. For example, NIRS requires a vibrational Hamiltonian, whereas X-ray absorption spectroscopy requires an electronic Hamiltonian. The frequency domain approach can involve applying a quantum phase estimation (QPE) circuit including performing the initial state preparation, performing the lineshape preparation (encoding the distribution of possible values of delay), applying a controlled time evolution, performing a quantum Fourier transform, and measuring, directly yielding frequency from distribution of interest. The time domain approach can involve applying a Hadamard-test circuit for different times, including initial state preparation and controlled time evolution, where the distribution of times to measure can be dictated by the lineshape, and the measurement yields statistics which are used for a classical Fourier transform to recover the spectrum.

In other words, encoding information pertaining to the distribution can be performed using an associated distribution of circuits encoding different time durations. In the frequency domain approach, all circuits are the same and they all encode the distribution fully, whereas in the time domain approach, the circuits are different.

There can be different orientations associated to the dipole operator. For instance, in cartesian coordinates, there can be the x, the y, and the z coordinate. In many cases, it will be relevant to consider more than one orientation. This can be performed by repeating the process described above with reference to Fig. 2 for more than one orientation. Indeed, in the step of preparing 114 the initial state, the dipole operator can encode the electromagnetic interaction in a target orientation. The time evolution considers a change between an initial and a final state. The final state can correspond to a time at which expectation values of the dipole operator in a final orientation are measured. In some instances of the process, such as when performing the process for NIRS or XAS, two examples of linear absorption spectroscopy, the target orientation and the final orientation will be the same, e.g., x, y or z, and the process can be repeated for one, two, or all three target orientations (typically all three). In such cases, in the 3 by 3 matrix associated to the dipole operator, only the diagonals are of interest. However, in other cases, such as the case of polarized photon absorption spectroscopy and EELS, two examples of angular-resolved spectroscopy, the former orientations may be useful but may not be sufficient to provide the whole picture, and "off diagonal" elements may need to be considered as well. The process may need to be repeated one, two, or three additional times, with the final orientation different from the target orientation. Maintaining the example of Cartesian coordinates, the process may need to be repeated in one, two or three additional times (typically all three), with i) the initial orientation as x and the final orientation as y, ii) the initial orientation as y, and the final orientation as z, and iii) the initial orientation as z and the target orientation as x, to name some possible examples. In angular-resolved spectroscopy scenarios, one or more additional ancilla qubits can be used to provide additional probing information which, during post processing, can allow resolving different angle detections.

More specifically, simulating photon absorption spectra and electron energy loss spectroscopy on quantum computers can rely on the framework of computing the "dynamic structure factor" of a target system which includes both the direction and the energy of the incoming probe. This requires computing dipole correlation function expectation values, $\left〈0\left|{U}_{{ζ}_{0}}^{†}{e}^{- iHt}{U}_{{ξ}_{0}}\right|0\right〉$, for each unique pair of Cartesian coordinates *ξ,ζ*. This amounts to 3 unique on-diagonal terms where *ξ* = ζ, and 3 unique off-diagonal terms, *ξ*≠ ζ.

In all cases, e.g., linear absorption spectroscopy cases averaged over orientations, and angular-resolved spectroscopy cases, an algorithm can include: 1) synthesizing a unitary circuit for each Cartesian coordinate of the dipole rotated ground state for initial state preparation, U_{ξ0}, and 2) computing on-diagonal terms xx, yy, and zz terms (examples will be provided below). In the cases where off-diagonal terms are to be considered, the following steps can further be used: 3) computing xy, xz, and zy terms using the modified Hadamard test algorithm presented below, and 4) classically determining a set of q vectors and combining all 6 expectation values to compute the spectroscopic signal.

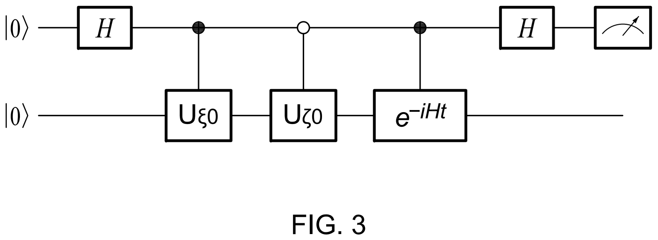

To compute the desired expectation value on a quantum computer a modified Hadamard test may be needed due to the cross terms with the circuit illustrated in Fig. 3, whereas in linear absorption spectroscopy cases, this circuit simplifies to a version provided further below. In this example, the circuit uses a linear combination of unitaries of the two state preparation circuits, *U*_{*ξ*0/*ζ*0} (of the dipole operator in one Cartesian direction applied to the ground state of the target system) with equal weights.

Note that in the case of targeting the 'off-diagonal' terms of the dipole matrix (e.g. xy), which of the two directions of the dipole is applied can be determined by using an ancilla qubit to apply in a controlled manner (e.g. ancilla on 0 (open control) -> prepare direction x; ancilla on 1 (control) -> prepare direction y). Accordingly, when processing for xy the initial state preparation step can be controlled on the additional ancilla qubit for closed control (i.e. controlled on 0) preparation of x and open control (controlled on 1) for preparation of y, after having prepared the ancilla qubit. The one or more additional ancilla qubits can provide additional probing information which during post processing via improved off-diagonal correlation function can be used to resolve the different angle detections.

Moreover, when processing "off-diagonal" terms using frequency domain implementations, it can be relevant to, between applying the time evolution and applying the Fourier transform, apply block encoding of the dipole in the target direction and apply a Hermitian conjugate of the circuit which was used to prepare the initial state to the system register, and measuring the system register.

It will be noted that both the time domain and the frequency domain approaches can rely on the Trotter approach to perform the controlled time evolution. Indeed, in many cases, the Hamiltonian cannot be diagonalized as a whole, so the Hamiltonian can be discretized into diagonalizable fragments, and a product formula can be used to approximate the time evolution performed by the time evolution operators. Discretizing the Hamiltonian leads to an error, and it can be difficult to tell what that error is, making it difficult to make a choice of time step which both limits the computational cost while also satisfying a suitable error. Indeed, it was found that relying on upper bounds of the error to determine the step size can be overly pessimistic, leading to a number of steps which can be a lot higher than necessary to achieve the desired error, making the quantum computations slow.

It was found that in some embodiments, the following process could yield a more suitable determination of step size:
a - Decompose the Hamiltonian into "fast-forwardable" (i.e., diagonalizable) fragments;
b - Set a product formula for approximating the time-evolution (e.g. second-order Trotter) - what formula to choose might depend on how to exploit the structure of the Hamiltonian fragments;
c - Use Baker-Campbell-Hausdorf (BCH) expansion to get leading-order error operator - a function that takes inputs a and b and outputs a leading-order error operator (higher-order terms can be ignored);
d - Obtain approximate classical solution of the Hamiltonian (pick the optimal solution that is can be realized computationally). This yields a series of approximate eigenstates;
e - Compute the target initial state as a linear combination of the approximate (classically obtained) eigenstates;
f - Calculate expectation values of BCH error operator for obtaining perturbative estimates of error in eigenstates of interest (e.g. those with large overlap with initial state)
   - Inputs:
      - BCH leading-order operator (from step c)
      - Approximate eigenstates (from step d)
      - Eigenstates with significant overlap with initial state (from step e)
      - Output: Expectation values from which evolution time can be determined; and
g - Specify target error (*ε_{ν}*) in eigenstates, obtaining evolution time *τⱼ* for each eigenstate of interest (see equation 35 below).

Accordingly, the time step required to keep the error under an error target can be calculated for each eigenstate. For the full simulation, the different eigenstates are to be considered. There are different ways of using information obtained so far. A conservative approach would be to use the minimum step out of all the ones that were obtained. However, not all eigenstates matter the same for the spectrum. By performing the following step h, more weight can be placed onto important eigenstates and ignore less relevant ones, which can have a significant effect on limiting computational costs.
h - Calculate weighted average of time-steps required for each eigenstate of interest (see equation 36)
   - Inputs:
      - *τⱼ*'s (from step g)
      - Decomposition of initial state into approximate eigenstates (from step e)
   - Outputs:
      - Evolution time (*τ* - see equation36)
i - Obtain the required number of steps for product formula to achieve target accuracy (see equation 37)

The approaches presented above can operate on a quantum computer including one or more classical computers and one or more quantum systems, where some initialization and post-processing steps can be carried out on the former, and the correlation function quantum modules can be carried out on the latter.

The quantum computer can be used for loading or preparing the initial state, obtained through classical means or described using a quantum circuit. The quantum computer can be composed of superconducting qubits, photonic qubits, trapped-ion qubits, silicon-based qubits, or neutral atoms, to name some examples. These devices apply gates that depend on the circuit used to prepare the initial state. This process can produce the desired statistics required to obtain the dynamical quantities of interest for the quantum system.

In other words, computing a leading-order error operator can include performing a Baker-Campbell-Hausdorf (BCH) expansion based on fragments of the Hamiltonian which is the subject of the quantum processing and which can be diagonalized and on a product formula approximating the time evolution performed by the time-evolution operators. The product formula has a formula time step, a formula Hamiltonian effectively implemented by the product formula, and product formula expectation values.

The computing of a set of eigenstates and eigenvalues is performed on an approximate classical solution of the Hamiltonian which is the subject of the quantum processing, and this can be performed on the best classical model that is computationally affordable, for instance. The eigenvalues can be ignored and focus can be placed solely on the eigenstates. This can be done in parallel with or before computing the leading-order error operator.

The computing the product formula expectation values of the leading-order error operator can be done for at least some (when some, only those that have a significant overlap with initial state, degree of overlap can be user-defined) eigenstates of the set (these constitute a perturbative estimate of the difference between the computed eigenvalues and eigenvalues of the product formula).

The determination of a maximum time step associated to a target error of a portion or all of the at least some eigenstates of the set can be done using the product formula expectation values (3).

Hamiltonian which is the subject of the quantum processing has target eigenvalues/eigenstates, the product formula has an associated Hamiltonian. If the product formula was perfect, the product formula Hamiltonian would equal the Hamiltonian which is the subject of the quantum processing, but the product formula is imperfect, causing an error. A measurement of the difference between the two, or how much error is introduced by the product formula, is thus obtained. This error will grow as a function of the time step in accordance with a relationship, but the exact relationship can change based on the specific problem.

In some embodiments, the spectrum can lie in a region of interest which is not the full range of the Hamiltonian. In such cases, removing large peaks can avoid wasting resources in simulating and measuring information that is outside the range of interest, and therefore allow a performance gain independently of whether the step size determination process described above is implemented or not. An example application is near-infrared spectroscopy, where 90 to 99.9% of spectrum lies in the mid-infrared region that is not of interest. Removing this component in this embodiment can effectively speed up the algorithm by a factor of 10²-10⁶.

To begin, a window of frequencies of interest where the spectrum is to be calculated can be determined. An approximate classical solution of Hamiltonian (same as step d of Trotter step size) can be performed. And the initial state can be represented as a linear combination of approximate eigenstates. Then, eigenstates outside the window of interest can be removed from the linear combination eigenstates. This can be performed by building and applying a projection operator on the initial state, or in any other suitable manner, such as setting the coefficients in the linear combination which correspond to eigenstates lying outside the window of interest to zero (filtering), for example, and the resulting, filtered, linear combination of eigenstates can then be renormalized. The initial state can then be prepared on the quantum computer based on the filtered and renormalized linear combination of eigenstates.

The window of frequencies can be determined in a way to provide for some added "padding", and in other words be somewhat larger than the window within which the frequencies of interest strictly lie, so that the spectrum is not affected. The size of the padding can depend on the level of confidence of how good the approximate solution is, taking into consideration how close the approximate energies are from exact ones. The confidence can depend on the type of problem. For instance, it can be known that and approximate solution to a vibrational problem gives eigenvalues within 100 wavenumbers of exact ones. Applying three standard deviations, for instance, a padding of 300 wavenumbers can be applied to the window of frequencies, maintaining information for up to 300 wavenumbers outside the window within which the exact frequencies of interest are known to lie.

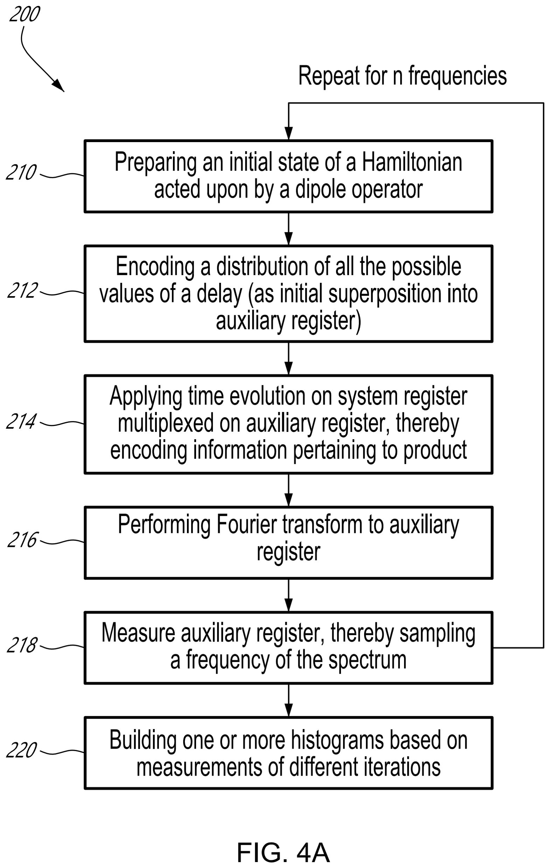

Fig. 4A presents an example of a frequency domain implementation of a method of determining or measuring 200 a spectrum stemming from an electromagnetic interaction with one or more molecules. In this example, a plurality of iterations is performed, each iteration including preparing 210 the initial state, encoding 212 the distribution, applying 214 the time evolution, performing 216 a Fourier transform, and measuring 218. Each measurement samples a frequency of the spectrum and the iterations thus sample different ones of the frequencies. One or more histograms (e.g., one for each orientation of interest, such as via performing a plurality of sets of iterations with different initial states) are built 220 based on the measurements acquired from the different iterations, and the spectrum is recovered from the one or more histograms.

In the frequency domain approach, all of the possible values of the delay are encoded at once as an initial superposition into an auxiliary register having a plurality of auxiliary qubits. The time evolution is applied on the system register multiplexed on the auxiliary register. The Fourier transform is applied in a quantum manner, on the auxiliary registers.

Building 220 the histogram can include integrating the measurement of the auxiliary register at the condition that measuring the system register confirms that the system register corresponds to the initial state. The histogram can be normalized based on the measurements acquired in each one of the iterations.

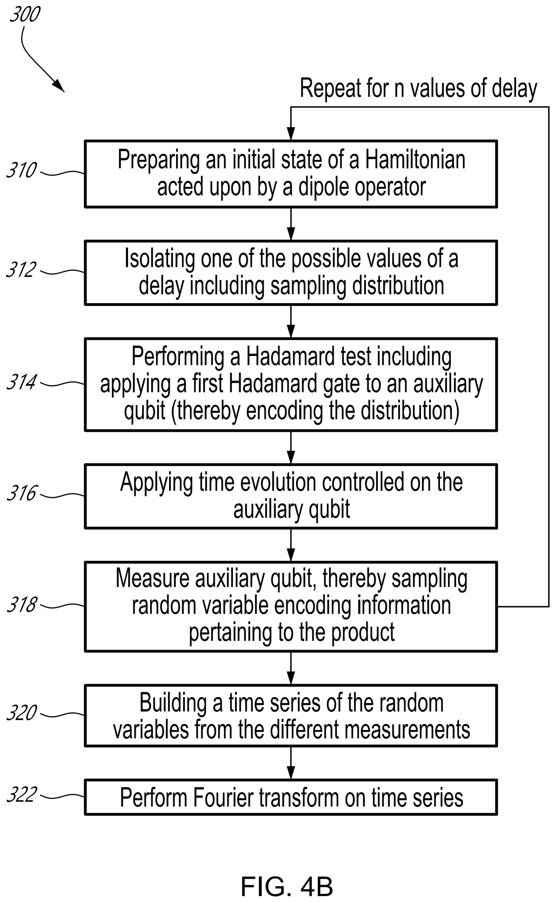

Fig. 4B presents an example of a time domain implementation of a method 300 of determining or measuring a spectrum stemming from an electromagnetic interaction with one or more molecules. In this example, a plurality of iterations are performed, each iteration including preparing 310 the initial state, isolating 312 one of the possible values of the delay distribution based on a sampling process (e.g., a Monte Carlo processor a shot allocation process), performing 314 a Hadamard test including applying a first Hadamard gate to an auxiliary qubit (which can be alternately referred to here as an ancilla qubit), applying 316 the time evolution controlled on the ancilla qubit, and measuring 318 the ancilla qubit, which samples a single random variable encoding information pertaining to a component of the product. The iterations can be repeated in a manner to cover different ones of the possible values, and typically multiple samplings of same ones of the possible values, and a time series can be built 320 based on the values measured at each iteration. A Fourier transform can then be performed 322 on the time series, from which the spectrum can be recovered.

In the frequency domain approach, a single value of the time delay is processed at each iteration, and the information pertaining to the distribution can be said to be encoded via the sampling, when taking all the iterations into consideration.

In practice the first Hadamard gate followed by the application of the time evolution can encode only a real part of the component of the product into the ancilla qubit, and a second Hadamard gate can be applied after applying a Hermitian conjugate of a phase gate on the ancilla qubit, followed by applying the time evolution and performing the measurement on the ancilla qubit, the application of the second Hadamard gate encoding the imaginary part of the component of the product into the ancilla qubit.

In the context of embodiments performed in accordance with the time domain approach, the Hadamard test circuit can be used to recover information from the spectrum. It was found that the Hadamard test could be modified to obtain additional information, adding more qubits in exchange for a lower number of circuit repetitions, which can be a suitable trade-off in many embodiments.

This modification can be as follows. First, a Hadamard test circuit such as shown in Fig. 3 can be performed, down to measuring the ancilla qubit. An additional register can be prepared with an additional wavefunction. A SWAP test can then be applied, using an extra ancilla qubit, between the ancilla qubit and the output of the Hadamard test circuit. The measurement yields additional information of expectation values of interest. The expectation value can then be obtained with a smaller number of circuit repetitions compared to only applying the Hadamard test. Accordingly, a greater accuracy may be obtained for a given number of iterations, or a target accuracy may be reached with a lesser number of repetitions.

A detailed example will now be provided with respect to near-infrared (NIR) radiation, and example performance gains will be presented. The performance gains are attributed to the general structure of the problem, and namely the fact that it is modeled under the "dipole approximation". Accordingly, performance gains would be expected for other types of spectroscopies which operate under the same model of dipole approximation, detailed more thoroughly above.

When NIR radiation is passed through a sample, the radiation is partially absorbed by the constituent molecules. Indeed, NIR radiation is energetically too weak to generate electronic excitations, as happens for example with UV-visible or X-ray spectroscopy: instead, incoming radiation is translated into vibrations of the atoms in the absorbing molecule. The strength of absorption at a particular frequency of incoming radiation, or more specifically the frequencies and intensities of the peaks, completely determine the absorption spectrum, which implies that the spectrum itself is a unique fingerprint of the molecule. Thus, if molecular spectra are cataloged a priori, it should be possible to detect the presence of a given molecule in an unknown sample through a direct comparison against the catalog. Chemical detection can be performed in this manner with mid-IR spectra. The peaks present in the mid-IR range (4,000-400 cm⁻¹) are fundamental vibrational excitations: there is usually no shortage of sharp, high-intensity features that are highly specific to a given molecule and thus can serve as good spectral fingerprints.

However, NIR spectroscopy (NIRS) has many advantages as an experimental technique, advantages that often make it more desirable than mid-IR for detection applications. In addition to being a low-cost and high-speed approach, weaker absorption means that it can be applied to much larger samples, as it is able to penetrate the bulk; for the same reason, NIRS is applicable to a much wider variety of phases of matter. At the same time, the ease of miniaturization of devices operating in the NIR part of the spectrum may allow for more portable detectors - most recently even reaching an individual consumer though cheap mobile phone attachments. On the instrumentation side, the telecommunications industry has commercialized many of the technologies that enabled cheap and reliable NIRS - chiefly among those the ability to use fiber optic cables for optic light transmission, an approach off-limits to mid-IR due to high signal attenuation in fiber optic in the associated spectral region. Finally, since less sample preparation may be required during spectrum acquisition, the method may be more non-destructive and non-invasive.

All these advantages make NIRS a desirable approach for chemical detection. Unfortunately, unlike their mid-IR counterparts, NIRS spectra typically feature broad distributions of intensity spread across many wavelengths, rather than sharp isolated features that would make it easy to identify specific molecules by a handful of key wavelengths. While the spectra are still unique to a given molecule, a correct identification requires considering the global intensity pattern rather than a few isolated signature peaks.

Accordingly, in the case of NIR spectra, identification via direct comparison to stored spectra may not be feasible.

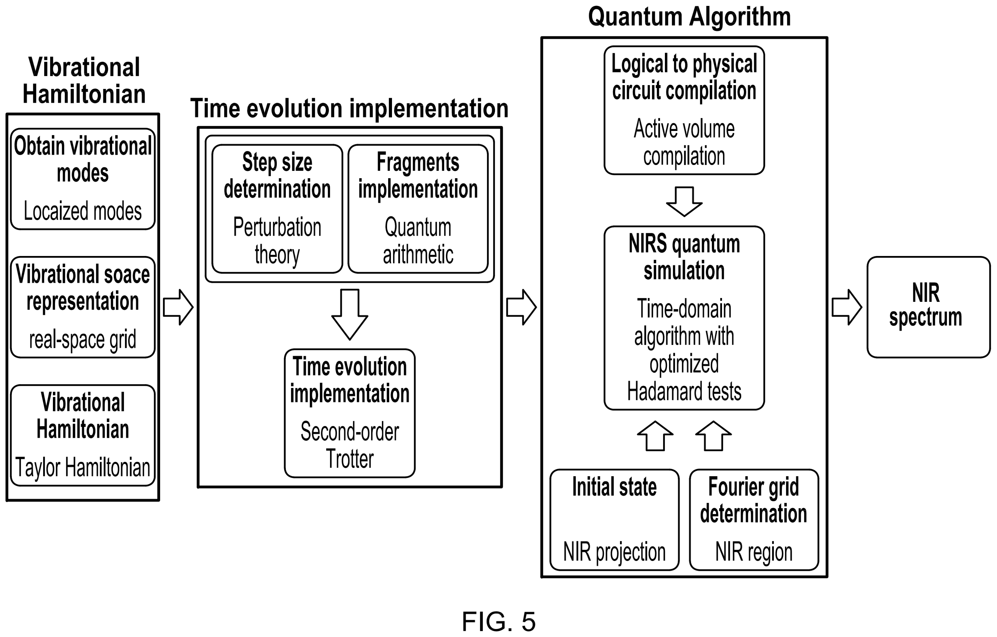

It was found that the NIR absorption spectrum in equation (1) can be obtained, in accordance with one example, using an algorithm which will now be described, with reference to Fig. 5. The algorithm, when performed on an appropriate initial state, can recover not only eigenvalues of a given Hamiltonian, but directly sample the absorption spectrum while also incorporating broadening effects. This algorithm can be performed directly in frequency space or with a time-domain approach that performs the Fourier transform on a classical computer. In this example a time-domain implementation is used.

In this example, a vibrational Hamiltonian representation based on the Taylor expansion of the PES is used, which will be referred to as the *Taylor form* for brevity. This choice enables working with a real-space representation, which can lead to improved cost estimates. Alternative representations will be presented below.

The starting point is the set of normal coordinates {*q*₁, ··· , *q_{M}*}, expressed in natural units, where each *qⱼ* is a scalar associated with a collective displacement of the atoms. Such coordinates are normally obtained by diagonalizing the Hessian of the PES near the equilibrium geometry. Then the harmonic component of the Hamiltonian is written as ${\hat{H}}_{h arm} = \frac{1}{2} {\sum }_{j = 1}^{M} {ℏω}_{j} \left({\hat{p}}_{j}^{2}+{\hat{q}}_{j}^{2}\right) ,$ (2) where *p̂ⱼ* is the momentum operator associated with *q̂ⱼ*, *ωⱼ* is the harmonic frequency of mode *j,* and M *=* 3*N*_{a*toms*} - 6 is the number of vibrational modes, with *M =* 3*N*ₐ*ₜₒₘₛ -* 5 for linear molecules. The position and momentum operators follow the canonical commutation relations [*q̂ⱼ*,*q̂ₖ*] *=* [*p̂ⱼ*,*p̂ₖ*] = 0 and [*q̂ⱼ*,*p̂ₖ*] = *iℏδⱼₖ*. The full vibrational Hamiltonian *Ĥ* is then written as $\hat{H} = {\hat{H}}_{h arm} + {\hat{H}}_{a nh} ,$ (3) where the anharmonic component can be expressed by a Taylor expansion of the PES with respect to the normal coordinates ${\hat{H}}_{a nh} = {\sum }_{i \geq j \geq k} {Φ}_{ijk}^{\left(3\right)} {\hat{q}}_{i} {\hat{q}}_{j} {\hat{q}}_{k} + {\sum }_{i \geq j \geq k \geq l} {Φ}_{ijkl}^{\left(4\right)} {\hat{q}}_{i} {\hat{q}}_{j} {\hat{q}}_{k} {\hat{q}}_{l} + ⋯ .$ (4)

The Taylor coefficients can be obtained from doing a fitting of the PES. The present disclosure refers to two attributes of a particular vibrational Hamiltonian: its maximum Taylor expansion order, which corresponds to the largest sum-total monomial power appearing in the expansion; and its *n*-mode character, where *n* is the maximum number of distinct modes appearing together in a monomial. As an example, a Hamiltonian *Ĥ* = *q̂*₁*q̂*₂*q̂*₃*q̂*₄ would have Taylor order four and be 4-mode, while $\hat{H} = {\hat{q}}_{1}^{2} {\hat{q}}_{2} {\hat{q}}_{3}$ would also have Taylor order four while only being 3-mode. In practice one often restricts the allowed inter-mode interactions to 3- or even 2-mode, while maintaining Taylor order of four: this corresponds to setting coefficients such as Φ*ᵢⱼₖₗ* or Φ*ᵢⱼₖₖ* to zero.

The most expensive subroutine in the process is the time-evolution operator *e*^{-*iĤt*}. The cost of this operation is directly related to the number of terms in the Taylor expansion in equation 4. Any techniques that permits reducing this number of terms while not hindering accuracy are particularly useful for reducing the overall cost of the quantum algorithm. The mode localization approach is used herein, which effectively makes the Hamiltonian more sparse and lowers the overall implementation cost. The usage of mode localization techniques has been shown to greatly reduce the required number of inter-mode interactions, with the example of the ethane C₂H₄ Hamiltonian yielding the same accuracy using a 2-mode representation with localized modes when compared to using a 4-mode expansion with (canonical) normal modes. By analogy to the use of localized orbitals in electronic structure calculations, mode localization redefines the vibrational modes *q̂ᵢ*'s so as to make the vibrations assume a more local character in the molecule. This is in contrast to the normal modes of vibration that are typically described by collective displacements of all atoms in a molecule. Mode localization yields a Hamiltonian of the form $\begin{matrix}\hat{H} = {\sum }_{i \geq j} \left({K}_{ij}{\hat{p}}_{i}{\hat{p}}_{j}+{Φ}_{ij}^{\left(2\right)}{\hat{q}}_{i}{\hat{q}}_{j}\right) + {\sum }_{i \geq j \geq k} {Φ}_{ijk}^{\left(3\right)} {\hat{q}}_{i} {\hat{q}}_{j} {\hat{q}}_{k} \\ + {\sum }_{i \geq j \geq k \geq l} {Φ}_{ijkl}^{\left(4\right)} {\hat{q}}_{i} {\hat{q}}_{j} {\hat{q}}_{k} {\hat{q}}_{l} + ⋯ ,\end{matrix}$ (5) the main difference being the introduction of non-diagonal momentum terms. The coordinates *q̂ᵢ* and coefficients Φ have been transformed, but for brevity the same symbols will be used and from now on refer exclusively to localized modes, unless explicitly stated otherwise.

Two alternative representations of the Hamiltonian can be considered. The most common representation is to pass from position and momentum operators *q̂,p̂* to the bosonic ladder operators b, *b*^{†}, re-expressing the higher-order potential energy surface terms like *q̂ᵢq̂ⱼq̂ₖ* as powers of these creation and annihilation operators. This is referred to as the bosonic representation. A different, relatively recent approach is to use the so-called Christiansen representation (sometimes also somewhat vaguely called the occupation-number representation), where the bosonic ladder of excitations for each mode is truncated and a new register is associated to each modal.

However, using the real-space representation can provide two significant benefits over these second-quantized approaches. First, the real-space form discretizes each vibrational mode with exponential accuracy with respect to the used number of qubits, which alongside the compact Taylor representation of the Hamiltonian yields significantly fewer terms when compared to using a second-quantized approach. More importantly, the real-space form allows the use of highly-optimized quantum arithmetic primitives (addition and multiplication) together with a phase gradient trick to implement time-evolution, resulting in a cheaper quantum algorithm overall.

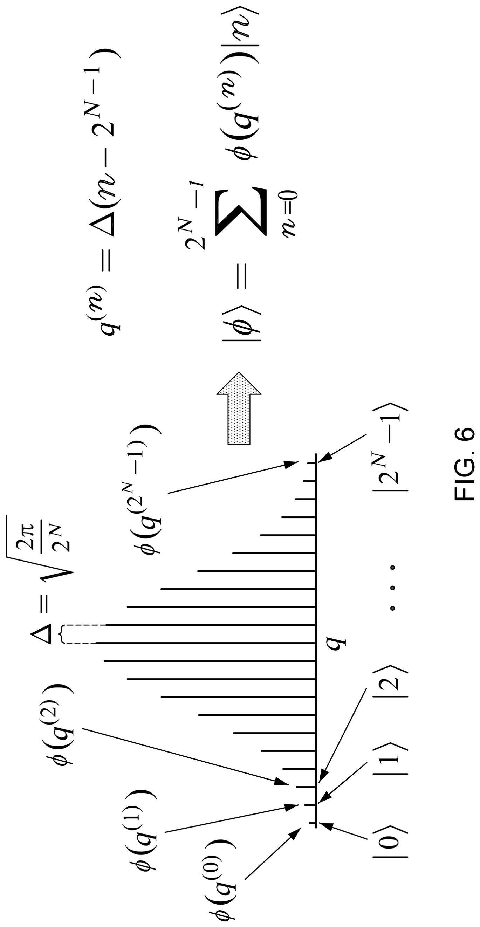

Once the Taylor expansion constants in equation (5) has been obtained, a qubit-based representation of the vibrational space needs to be defined. The real-space representation is adopted. The basic idea is to have a qubit register consisting of *N* qubits for each vibrational mode. For a given mode *q*, each of the computational basis states then encodes a point in a uniform grid that is associated to the discretization of *q* using 2*^{N}* grid points as shown in Fig. 6. From this there are 2*^{N}* different computational basis states for each vibrational mode: the *n*-th point in the grid for a vibrational mode *q* has an associated computational basis state such that $\hat{q} \left|n\right〉 = Δ \left(n-{2}^{N - 1}\right) \left|n\right〉 ,$ (6) where the constant $Δ ≡ \sqrt{2 π / {2}^{N}}$ is defined with an associated total grid width of $\sqrt{π {2}^{N + 1}}$. The number of qubits *N* required for achieving a faithful representation of vibrational excitations consisting of up to *N* quanta in this grid scales logarithmically with respect to *N.* This exponential accuracy with respect to *N* can be understood via the Nyquist-Shannon sampling theorem, and effectively allows for accurate encoding of the vibrational space with a small number of qubits. Applying the QFT on a given mode's qubit register is then simply a change of basis between the position and momentum representations: the transformation has the form $\hat{p} = {QFT}^{†} ⋅ {\hat{X}}_{s} ⋅ \hat{q} ⋅ {\hat{X}}_{s} ⋅ QFT ,$ (7) where *X̂ₛ* corresponds to an *X* gate on the most significant qubit of the register encoding the vibrational mode *q.* This *X* gate effectively performs a "shift" in the same way as usually done for the discrete Fourier transform in classical computing, which effectively centers the Fourier transform in some range [-*a*, *a*) instead of [0,2*a*). As such the combination *X̂ₛ* · QFT can be referred to herein simply as the shifted QFT.

Having obtained the vibrational Hamiltonian, it can be used to obtain its absorption spectrum as shown in equation 1.

When using an appropriate input state, QPE directly samples frequencies from the associated absorption spectrum. When applied to the normalized initial state $\left|{ψ}_{ρ}\right〉 = \frac{{\hat{μ}}_{ρ} \left|{E}_{0}\right〉}{\left|{\hat{μ}}_{ρ}\right|} ,$ (8) where the short-hand |*µ_{ρ}*| = ∥*µ̂_{ρ}*|*E*₀〉∥ is used, the canonical QPE procedure returns the frequency-dependent distribution ${P}_{ρ} \left(ω\right) = \frac{1}{{\left|{μ}_{ρ}\right|}^{2}} {\sum }_{f} {\left|\left〈{E}_{f}\left|{\hat{μ}}_{ρ}\right|{E}_{0}\right〉\right|}^{2} L \left(ω-{E}_{f}\right) .$ (9)

The lineshape function 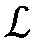 (*ω*) is linked to the initial preparation of the time/frequency register.

How this quantity can be expressed as a Fourier series is shown below. Before the addition of the lineshape, the similar Fourier integral can be written as: $\begin{matrix}{Z}_{ρ} \left(ω\right) ≡ {\int }_{- ∞}^{∞} {dte}^{iωt} \left〈{ψ}_{ρ}\left|{e}^{- i \hat{H} t}\right|{ψ}_{ρ}\right〉 \left(10\right) \\ = 2 π {\sum }_{f} {\left|\left〈{E}_{f}\left|{\hat{μ}}_{ρ}\right|{E}_{0}\right〉\right|}^{2} δ \left(ω-{E}_{f}\right) . \left(11\right)\end{matrix}$ (11)

From this the target distribution may be obtained as ${P}_{ρ} \left(ω\right) = \frac{1}{2 π {\left|{μ}_{ρ}\right|}^{2}} {Z}_{ρ} \left(ω\right) ∗ L \left(ω\right) ,$ (12) where * defines the convolution operation. Using the convolution theorem, and considering that a Lorentzian lineshape is targetted such that 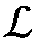 (*ω*) *= η*/(*ω*² + *η*²) with associated Fourier conjugate *e*^{-*η*|*t*|}/2, it can be shown that ${P}_{ρ} \left(ω\right) = \frac{1}{2 π {\left|{μ}_{ρ}\right|}^{2}} {\int }_{- ∞}^{∞} {dte}^{iωt} \frac{{e}^{- η \left|t\right|}}{2} \left〈{ψ}_{ρ}\left|{e}^{- i \hat{H} t}\right|{ψ}_{ρ}\right〉 .$ (13)

The next step is to approximate the integral on the right-hand side of equation (13) to some accuracy *ε* by truncating the infinite time as ${\int }_{- ∞}^{∞} \to {\int }_{- {T}_{m ax}}^{{T}_{m ax}}$, which can be done by choosing ${T}_{m ax} = \frac{1}{η} log \frac{1}{ϵ} .$ (14)

This finite integral can now be expressed using a Fourier series. Noting that *P_{ρ}*(*ω*) is localized inside of some spectral range [*ω*_{m*in*}, *ω*_{m*ax*}], a finite Fourier series can be used to expand *P_{ρ}*(*ω*), effectively making it periodic outside of this range. Defining the spectral width as Ω = *ω*_{m*ax*} - *ω*_{m*in*}, this effectively defines a Fourier grid for a discrete Fourier transform. The Fourier series approximation of the target distribution can be obtained as: ${P}_{ρ} \left(ω\right) \approx \frac{1}{Ω {\left|{μ}_{ρ}\right|}^{2}} \left[\frac{1}{2}+{\sum }_{k = 1}^{{k}_{m ax}} Re \left({p}_{ρ}\left[k\right]{e}^{2 πikω / Ω}\right)\right]$ (15) where the number of Fourier coefficients is ${k}_{m ax} = \left⌈Ω⋅{T}_{m ax}\right⌉$ (16) and the Fourier components can be defined as: ${p}_{ρ} \left[k\right] = {e}^{- 2 πηk / Ω} \left〈{ψ}_{ρ}\right| {e}^{- 2 πi \hat{H} k / Ω} \left|{ψ}_{ρ}\right〉 .$ (17)

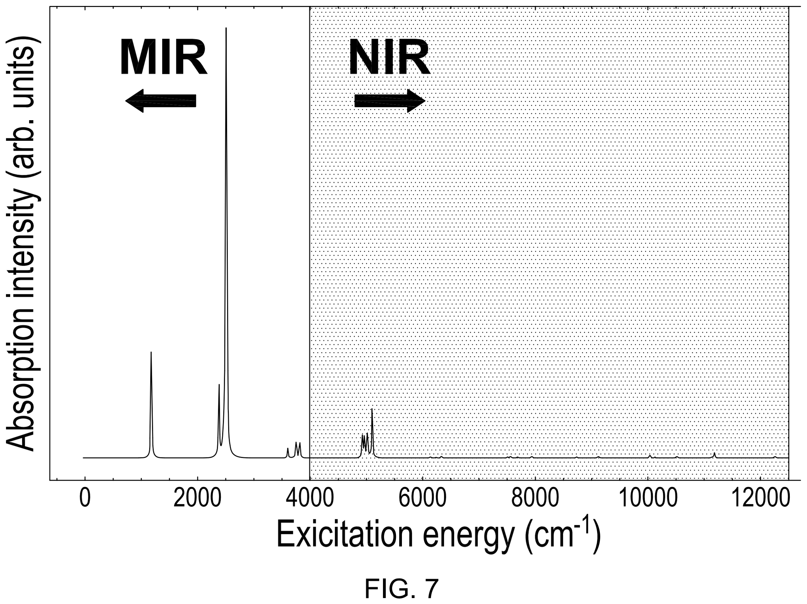

The unitary that is entering the expectation value is *e*^{-2*πiĤk*/Ω}, which can also be thought as rescaling the Hamiltonian *Ĥ* → *Ĥ*/Ω to effectively estimate the phase of *Ĥ*/Ω. Typically a rescaling factor Ω ~ 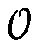 (∥*Ĥ*∥⁻¹) is made in order to keep the spectrum in the [-1,1] range and avoid aliasing in the Fourier transform that comes from making the *P_{ρ}*(*ω*) function periodic when approximating it as a Fourier series. However, this example focuses in the NIR spectrum, which consists of the range of frequencies between 4,000 and 12,500 cm ⁻¹. As seen in Fig. 7, the mid-IR (MIR) region compromises the largest absorption peaks, while the NIR region has peaks coming from overtones and combination bands that quickly decay as the excitation energy increases, while no significant vibrational excitations happen beyond this region. Since the interest is in the NIR region, it becomes unnecessary to simulate the mid-IR portion. Still, if the Fourier frequency window is chosen to be only in the NIR range, the non-zero spectrum coming from mid-IR would appear as an aliasing effect due to the periodic nature of the Fourier series. In order to avoid simulating any peaks from this region, a filtering procedure may be applied over the initial state |*ψ_{ρ}*〉, which can be written in the eigenstate basis as $\left|{ψ}_{ρ}\right〉 = {\sum }_{k} {a}_{k}^{\left(ρ\right)} \left|{E}_{k}\right〉 .$ (18)

The approach may be conceptualized as setting all ${a}_{k}^{\left(ρ\right)}$ to zero for which *ω*_{*k*0} *<* 4,000*cm*⁻¹, where the excitation energy is defined as *ω*_{*k*0} ≡ *Eₖ - E*₀. However, in practice one may be unable to have access to the exact eigenstates and energies. This may be circumvented by considering some approximate eigenstates |*Ẽₖ*〉, which in some embodiments of the present disclosure, are obtained from the mean-field method VSCF. The wavefunction can then be expanded in this basis as $\left|{ψ}_{ρ}\right〉 = {\sum }_{k} {{a}^{˜}}_{k}^{\left(ρ\right)} \left|{{E}^{˜}}_{k}\right〉$. Due to the approximate nature of these eigenstates and in order to avoid removing components that could appear in the NIRS spectrum, an energy cutoff value of *W* = 3,750 cm ⁻¹ is chosen, noting that VSCF energies typically approximate the exact energies in the mid-IR region within ~ 100 cm ⁻¹. By considering the projector ${\hat{Π}}_{W} = {\sum }_{k \left|{{ω}^{˜}}_{k 0}\geq W\right.} \left|{{E}^{˜}}_{k}\right〉 \left〈{{E}^{˜}}_{k}\right| ,$ (19) the (re-normalized) initial state Π̂*_{W}*|*ψ_{ρ}*〉 can be sent to the process to focus on the NIR region. Based on the foregoing, one may chose to simulate the window in the spectral range between *ω*_{m*in*} = 3,500*cm*⁻¹ and *ω*_{m*ax*} = 12,500 cm ⁻¹ to minimize the absorption line coming from the mid-IR region appears as an aliasing component in the recovered spectrum. This yields the spectral width Ω = 9,000*cm*⁻¹.

Besides allowing the simulation of the NIR region with the smaller Ω being related to smaller *k*_{m*ax*} and maximum evolution times, the removal of these states renormalizes the initial wavefunction, effectively boosting the overall accuracy in the NIR region by a factor of ∥Π̂*_{W}*|ψ*_{ρ}*〉∥². This can manifest as a large improvement in the accuracy requirements by around two to six orders of magnitude: the *µ̂_{ρ}*|*E*₀〉 optical excitation is typically constituted by 90 to 99.9% of states coming from the mid-IR region.

Using a product formula implementation of the time-evolution operator can be more useful than using a qubitization-based framework in this example. Being able to focus the spectrum in this particular NIR region cannot be done when using qubitization inside of a QPE framework. Qubitization necessitates the rescaling of the entire spectrum of *Ĥ* to the range [-1,1] through the appearance of the 1-norm *λ*, where the operator *Ĥ*/*λ* is effectively implemented as to keep the block-encoding unitary. However, in practice most of the absorption in this rescaled spectrum will be trivially zero, which is the property being exploited in this work. This happens since the representation used for the Hamiltonian will have many states that are never excited but with extremely high energies. An example of such a state would correspond to having all the amplitude of a given state in the real-space representation appearing only in the borders of the grid. From this, it may be discerned that as the system size grows and more of these states become available, *λ* will quickly increase, whereas the chosen Ω has a constant value of 9,000*cm*⁻¹ associated with the NIR region, which is the only region where there will be some absorption after the state projection procedure. Rescaling by Ω instead of *λ* then enables a vastly increased resolution in the energy x-axis. This consideration makes Trotter-based implementations of QPE-based algorithms for spectroscopy particularly attractive.

Having shown how to express the target distribution as a Fourier series with associated coefficients *p_{ρ}*[*k*] in equation (17), these can now be determined for each of *k =* 1, ··· , *k*ₘₐₓ. The *e*^{-2*πηk*/Ω} factor makes contributions from large k's vanishingly small, which in turn means that there is no need to determine the associated *p_{ρ}*[k]'s to the same high accuracy as those coming from smaller k's.

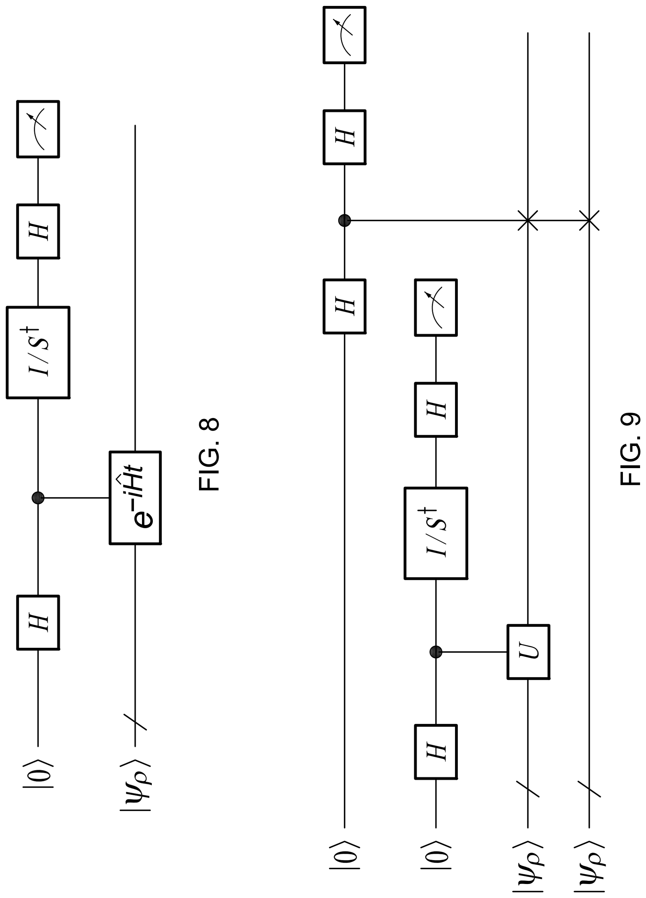

The key component of the time-domain formulation of the algorithm is the expectation value of the self-correlation function〈*ψ_{ρ}*|*e*^{-*iĤt*}|*ψ_{ρ}*〉. Shown in Fig. 8 is the Hadamard test quantum circuit for recovering this quantity through the implementation of the time-evolution for a given time t: the ancilla qubit encodes the expectation value of interest as ${p}_{0} \left(t\right) - {p}_{1} \left(t\right) = Re \left[\left〈{ψ}_{ρ}\right|{e}^{- i \hat{H} t}\left|{ψ}_{ρ}\right〉\right] .$ (21)

The imaginary component can be obtained instead if the gate in the Hadamard test after the controlled time-evolution is *S*^{†} instead of *I.* Here *p*₀(*t*) and *p*₁(*t*) are the probabilities of observing the ancilla in state 0 and 1 respectively for the controlled time-evolution *e^{-iĤt}*.

Considering a total number of shots 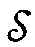 , one can then run the Hadamard test circuit in Fig. 8 for a time *t* = 2*πk*/Ω a total of ${N}_{k} = SN {e}^{- 2 πηk / Ω}$ (22) times for both the real and imaginary components, where $N = {\left[2{\sum }_{k = 1}^{{k}_{max}} {e}^{- 2 πηk / Ω}\right]}^{- 1}$ is a normalization constant. The m-th pair of real/imaginary measurements of the ancilla qubit with associated *k = kₘ* then yields the numbers ${x}_{ρ}^{\left(m\right)}$ and ${y}_{ρ}^{\left(m\right)}$ such that $\left〈{ψ}_{ρ}\left|{e}^{- 2 πi \hat{H} k / Ω}\right|{ψ}_{ρ}\right〉 = E \left[{x}_{ρ}^{\left(m\right)}+{iy}_{ρ}^{\left(m\right)}\right]$ *.* The non-uniform sampling over k's then incorporates the lineshape contribution to the spectrum, recovering the target spectrum as ${P}_{ρ} \left(ω\right) \approx \frac{1}{Ω {\left|{μ}_{ρ}\right|}^{2}} \left[\frac{1}{2}+\frac{1}{{N}_{0}}{\sum }_{m = 1}^{S / 2} Re \left[\left({x}_{ρ}^{\left(m\right)}+{iy}_{ρ}^{\left(m\right)}\right){e}^{2 πikω / Ω}\right]\right] ,$ (23) where 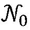 corresponds to considering equation (22) for k = 0. Some approaches have been proposed for post-processing this quantity by using, e.g., a matching pursuit algorithm, which could lower the overall required number of samples for achieving a target accuracy. However, here, no post-processing was done. Determination of 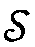 is purely a signal processing problem, corresponding to the accuracy that is required for the coefficients of a Fourier series to recover the associated signal to some accuracy, with the main quantities that determine the signal being the lineshape width *η* and the spectral range Ω. In practice, the quantity 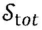 = 3 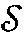 may be used to account for all three Cartesian components of the dipole appearing in the absorption spectrum. Practical choices of 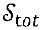 are obtained heuristically.

A sub-process which will be referred to here as the double measurement can involve re-using the output state after applying the Hadamard test to gain additional information of the associated expectation value. By performing a SWAP test on the output of the Hadamard circuit in Fig. 8, the amount of information that is recovered from each Hadamard test can be increased in exchange for having additional qubit registers. The circuit for performing this can be seen in Fig. 9. The usefulness of this approach can be seen by noting that the output state of the Hadamard test corresponds to $\frac{1}{2} \left(\hat{I}\pm \hat{U}\right) \left|{ψ}_{ρ}\right〉 ,$ (24) where the ± sign is determined by the outcome from measuring the Hadamard test ancilla qubit. The probability of measuring 0 on the extra ancilla from the SWAP test then corresponds to ${p}_{0} = \frac{1}{2} \left(1+{\left|\frac{1}{2}\left〈{ψ}_{ρ}\left|\hat{1}\pm \hat{U}\right|{ψ}_{ρ}\right〉\right|}^{2}\right) .$ (25)

The overall number of shots can be reduced by a factor *ζ* ∈ [1.22,2.37], the factor being determined by the expectation value being measured - with an average reduction of *ζ* = 1.69.

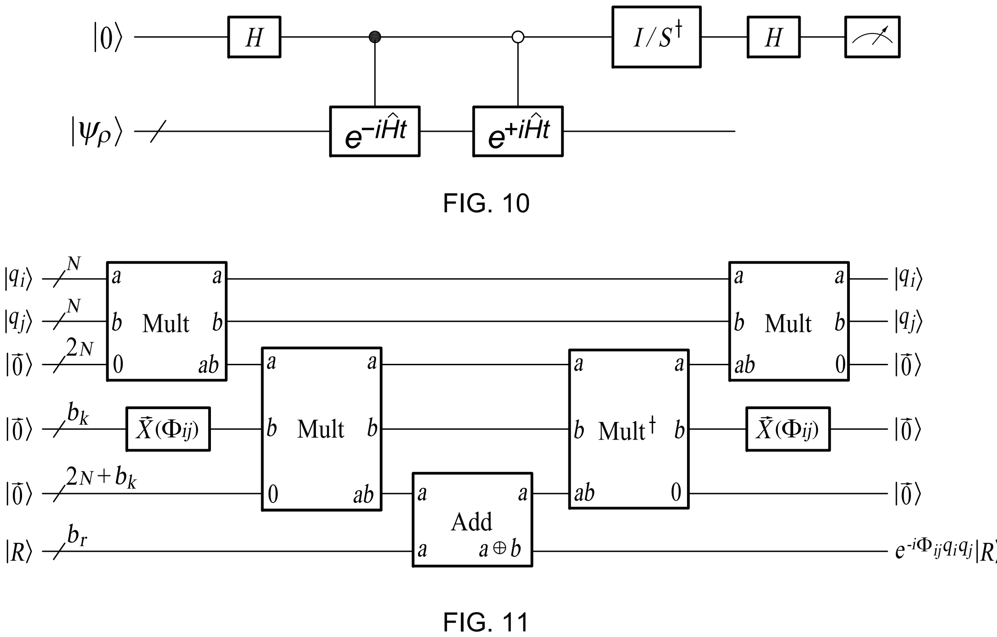

Another subroutine which will be referred to herein as the double phase method can optionally be implemented. The double phase method can change the controlled unitary that the Hadamard test implements as $\left[\begin{matrix}\hat{1} & 0 \\ 0 & {e}^{- i \hat{H} t}\end{matrix}\right] \to \left[\begin{matrix}{e}^{i \hat{H} t} & 0 \\ 0 & {e}^{- i \hat{H} t}\end{matrix}\right] ,$ (26) as shown in Fig. 10. The benefit of doing this is that the ancilla qubit now encodes the expectation value of *e*^{*-*2*iĤt*} instead of the *e^{-iĤt}* term seen in (21), which implies recovering the expectation value for an evolution time that is twice as long. Moreover, implementing the unitary corresponding to equation (26) can be done for an even lower cost than the controlled time-evolution in the original Hadamard test. To understand this improvement, one may start by considering the original Hadamard test, for which controlling the time-evolution simply necessitates controlling the addition operation to the circuit shown in Fig. 11. The coefficients coming from the Taylor expansion, e.g. ${Φ}_{ij}^{\left(2\right)}$, are encoded in a quantum register, with the associated ${e}^{i {Φ}_{ij}^{\left(2\right)} {\hat{q}}_{i} {\hat{q}}_{j}}$ operator then implemented using a phase kickback technique. Thus, instead of having to control the addition operation, the unitary in equation (26) for the modified Hadamard test can be implemented by simply flipping the qubit that encodes the sign of the ${Φ}_{ij}^{\left(2\right)}$ coefficient, which is done using a CNOT gate. When also considering the uncomputation cost (see Fig. 11) the modified unitary in equation (26) can be implemented with the same cost of the uncontrolled application of *e^{-iĤt}*, plus 2*N*_{T*aylor*} CNOT gates, where *N*_{T*aylor*} is the total number of non-zero coefficients appearing in the Hamiltonian in equation (4). This basically allows implementation of the required time-evolutions for approximatley half the computational cost.

Another technique can optionally be used, referred to herein as the active volume compilation technique. At an abstract algorithmic level, it can be thought of as reducing the time and resources needed to run quantum algorithms on a specific error-corrected hardware architecture. The basic idea is based on the observation that during typical computations, most of the qubits at any given time are idle. However, the computation cost is usually determined by the circuit volume, this being the number of qubits multiplied by the number of non-Clifford gates. By using additional qubits to compute chunks of logical operations that can be parallelized, these can then be applied via teleportation. The active volume is then determined by the number of logical operations making up these chunks, where each particular logical operation has an associated active volume that is measured in so-called *blocks.*

The overall cost of the computation is then dictated by the number of blocks instead of the circuit volume, which typically leads to one or more orders of magnitude reduction of the overall computation time. Note that the active volume compilation also already incorporates error correction for fault-tolerance through the surface code. In addition, the required teleportations benefit from having a hardware with high connectivity, making it particularly promising for certain photonic-based architectures. The usage of this technique for runtime optimization and estimation reflects the kind of techniques that could be used to compile the algorithms described herein on a physical level given that the specific technique and associated estimates would need to be tailored to the specific hardware under consideration.

The active volume compilation divides the qubits into two groups: working qubits and memory qubits. Memory qubits are used to accelerate the calculation by parallelizing blocks and storing intermediate results or ancillary states which are used in subsequent steps of the computation. The speed-up from the active volume can be estimated through the following steps:
1. Identify the required number of logical qubits for implementing a circuit of interest *Q_{L}.* For a given budget of logical qubits *Q_{B}*, set the number of working qubits as ${Q}_{W} = \left⌊{Q}_{B}/2\right⌋$ . Note that the active volume compilation technique requires that *Q_{W}* ≥ *Q_{L}*, thus at least duplicating the required number of logical qubits. The memory qubits are then allocated as *Q_{M} = Q_{W}.*
2. Calculate the active volume in blocks B for the quantum algorithm of interest. This is done by first decomposing the algorithm into elementary subroutines, which are in turn decomposed into a block-based implementation.
3. Divide the number of blocks by the number of memory qubits: this will approximate the required number of cycles to implement all operations as *N_{cycles} = B*/*Q_{M}.* Note that the block-based implementation of the circuit already includes error correction through the use of a surface code, making the associated improvement unable to be converted back to elementary logical gates.
4. Divide the number of cycles by the clock-rate *ν_{clock}* to obtain the total runtime of the circuit *T*ᵣ*ᵤₙₜᵢₘₑ = N_{cycles}*/*ν*_{c}*_{lock}.*

It should be emphasized that the above constitutes a high-level description of the procedure.

Having described both the quantum algorithm and the qubit mapping of the vibrational degrees of freedom, now the implementation of the time-evolution operator *e^{-iĤt}* and the circuit for initial state preparation corresponding to |*ψ_{ρ}*〉 in equation (8) is described. This initial state can be efficiently prepared at the cost that is negligible compared with that of implementing the time-evolution. This can be understood since a small number of mean-field VSCF states can be used to represent the initial state: the initial state is extremely sparse in this basis. Given that, the focus shifts to the implementation of the time-evolution operator. Start by separating the Hamiltonian into its kinetic and potential components *Ĥ* = *T̂* + *V̂*, where $\hat{T} = {\sum }_{i \geq j} {K}_{ij} {\hat{p}}_{i} {\hat{p}}_{j} = {{Q}_{M}}^{†} \left({\sum }_{i \geq j} {K}_{ij} {\hat{q}}_{i} {\hat{q}}_{j}\right) {Q}_{M}$ (27) $\begin{matrix}\hat{V} = {\sum }_{i \geq j} {Φ}_{ij}^{\left(2\right)} {\hat{q}}_{i} {\hat{q}}_{j} + {\sum }_{i \geq j \geq k} {Φ}_{ijk}^{\left(3\right)} {\hat{q}}_{i} {\hat{q}}_{j} {\hat{q}}_{k} \\ + {\sum }_{i \geq j \geq k} {Φ}_{ijkl}^{\left(4\right)} {\hat{q}}_{i} {\hat{q}}_{j} {\hat{q}}_{k} {\hat{q}}_{l} + ⋯ ,\end{matrix}$ (28) where the multidimensional shifted QFT is defined as the product of shifted QFTs over all modes: ${Q}_{M} = {\prod }_{j = 1}^{M} {\hat{X}}_{s , j} ⋅ {QFT}_{j} .$ (29)

The operator *V̂* is already diagonal in the real-space representation, while conjugation with Q_{M} makes the kinetic term diagonal. A second-order product formula can then be used to approximate the time-evolution as ${e}^{- i \hat{H} t} \approx {e}^{- i \hat{V} \frac{t}{2}} {e}^{- i \hat{T} t} {e}^{- i \hat{V} \frac{t}{2}} ≡ {\hat{U}}_{2} \left(t\right) .$ (30)

In an illustrative embodiment, a second-order Trotter formula is chosen since it was associated with the lowest implementation costs for achieving the required accuracy in the test systems when compared to other Trotter orders. The required evolution time for the unitary corresponding to a single time step, namely e^{-*iĤ*Δ*t*}, is not too long. This makes the required Trotter evolution time relatively short, which tends to benefit lower order Trotter formulas. to the term 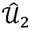 (*t*) is referred to as the Trotter oracle.

One approach resides in bounding the error to estimate the associated Trotterization cost that achieves a target accuracy. However, the resulting upper bounds to the Trotter error can be overly pessimistic, with tighter estimates such as direct simulations requiring a prohibitive amount of classical resources to be obtained. One of the main contributions of the present disclsoure is an alternative, cheap-to-implement approach to more tightly control the Trotter error: instead of aiming to upper-bound it, it is estimated using an approach based on perturbation theory. Then the step size can be determined based on this estimation. This tighter estimate is in turn used to obtain a less pessimistic step size for the Trotterized time-evolution, strongly reducing the associated cost of implementing the time-evolution operator. The approach can be extended to include excited states and consider a second-order Trotter formula. Still, all the discussion that follows can be straightforwardly extended to arbitrary-order Trotter formulas.

Recall that the algorithm for spectroscopy can be considered, to an extent, as performing phase estimation on the operator *Ĥ*Δ*t* for Δ*t* = 1/Ω. However, the Trotterized time-evolution operator effectively implements a time evolution under an effective Hamiltonian, namely *e*^{*-iĤeff*Δ}*^{t}.* The eigenvalues of *H_{eff}* that contribute to the absorption spectrum need to be made to be the same as those of *Ĥ* within some target accuracy *ε_{ν}*. To achieve this, start by partitioning the Trotterized time-evolution into r steps, from which can be written as: ${e}^{- i {\hat{H}}_{eff} Δ t} = {\left[{\hat{U}}_{2}\left(\frac{Δ t}{r}\right)\right]}^{r} .$ (31)

Using the Baker-Campbell-Hausdorff formula,it is derived that: ${\hat{H}}_{e ff} = \hat{H} + {\left(\frac{Δ t}{r}\right)}^{2} {\hat{E}}_{2} + O \left(\frac{Δ {t}^{4}}{{r}^{4}}\right) .$ (32)

Here, the error operator is defined as: ${\hat{E}}_{2} = \frac{1}{24} \left(2\left[\hat{T}, \left[\hat{T}, \hat{V}\right]\right]+\left[\hat{V}, \left[\hat{T}, \hat{V}\right]\right]\right) ;$ (33) different fragmentation techniques for the Hamiltonian may yield different error operators depending also on nested commutators. In the above, the straightforward fragmentation into kinetic and potential energy fragments as shown in equation (30) is adopted. Perturbation theory can be used to estimate the eigenvalues of *Ĥ_{eff}.*

The explicit dependence on Δ*t*/*r* will make different degrees of perturbation theory be associated with different powers of *Δt*/*r.* First order perturbation theory is enough in this case to obtain the leading order. Thus, the eigenvalues of *Ĥ_{eff}* may be obtained as: ${E}_{j}^{\left(eff\right)} = {E}_{j} + \frac{Δ {t}^{2}}{{r}^{2}} \left〈{E}_{j}\right| {\hat{E}}_{2} \left|{E}_{j}\right〉 + O \left(\frac{Δ {t}^{4}}{{r}^{4}}\right) ,$ (34) which deviate from the true eigenvalues depending on the chosen step size r. For simplicity, the time step size is defined as *τⱼ* = *Δt*/*r* such that the associated error is within some threshold, namely $\left|{E}_{j}^{\left(eff\right)}-{E}_{j}\right| < {ϵ}_{ν}$. Note that the Δ*t*/*r* factor is not always smaller than one for the chosen Δ*t* and r's in the present disclosure, meaning in principle higher-order contributions need to be considered. However, choosing r as to make the leading order error smaller than *ε_{ν}* causes the remaining terms to be small as well, as long as *ε_{ν}* is not too large. Reasonable choices of *ε_{ν}* yield high-quality spectra. From this, it is obtained that: ${τ}_{j} = \sqrt{\frac{{ϵ}_{ν}}{\left〈{E}_{j}\left|{\hat{E}}_{2}\right|{E}_{j}\right〉}} .$ (35)

The physical interpretation of *τⱼ* is as the largest time step that can be taken with the Trotterized time-evolution such that eigenvalue ${E}_{j}^{\left(eff\right)}$ of the effective Hamiltonian is within the allowable error threshold *ε_{ν}* from the true eigenvalue *Eⱼ*. Since the initial state given in equation (18) only has significant contributions from a small number of eigenstates and the final target quantity is the associated absorption spectrum, only the eigenstates making up the initial state needs to be taken into consideration such that errors in the energies of optically inactive states will not affect the quality of the spectrum. Each of the eigenstates with a weight associated to its overlap with the initial state |*ψ_{ρ}*〉 in equation (8) may be chosen, namely $τ = {\sum }_{j} {\left|\left〈{E}_{j}|{ψ}_{ρ}\right〉\right|}^{2} {τ}_{j} ,$ (36) where, for simplicity, the same *τ* is used for all three Cartesian components *ρ = x, y, z* by averaging the associated *τ*'s. All of these expressions are in terms of exact eigenstates, which are obviously not accessible for systems of interest. To circumvent this, approximate eigenstates can be used as done in the initial state projection procedure.

Once *τ* has been obtained, the associated number of Trotter steps can be recovered as $r = \left⌈\frac{Δ t}{τ}\right⌉ .$ (37)

The inclusion of the overlap factor in equation (36) makes it unnecessary to calculate the expression in equation (36) for all j's: it only needs to be evaluated whenever there is a significant overlap |〈*Eⱼ*|*ψ_{ρ}*〉|². The expectation values corresponding to 〈*Eⱼ*|*ε̂*₂|*Eⱼ*〉 can be efficiently approximated by using states obtained from VSCF and representing the *T̂* and *V̂* operators using bosonic ladder operators. The expectation value computed in this way might differ from one expressed in the real-space basis, because the commutator algebra of the discrete operators *q̂ⱼ,p̂ⱼ* agrees with that of the infinite-dimensional bosonic operators *bⱼ,* ${b}_{j}^{†}$ only on a finite low-energy subspace. However, as long as enough points were used to discretize the vibrational modes when building the real-space representation, this low-energy subspace of agreement encompasses the optically active region of interest. This condition was already met when discretizing the real-space grid.

Next, implementation of the exponential of a polynomial of *q̂ⱼ* operators is described. This allows all terms to be implemented in the Trotter formula with the use of Q_{M} to rotate between position and momentum bases. The basic idea of the latter is to use a resource state $\left|R\right〉 = {\sum }_{j = 0}^{{2}^{{b}_{r}} - 1} {e}^{{ijϕ}_{{b}_{r}}} \left|j\right〉 ,$ (38) which is defined over an *bᵣ*-qubit register with *ϕ_{bᵣ} =* 2*πℓ*/2*^{b^{r}}.* Here ℓ is an integer that carries the scale information, namely to what real numbers the integers n that will be added to this state correspond to. It can be easily shown that using a modular quantum addition circuit to add an integer n to this state then yields the phase $\left|n\right〉 ⊕ \left|R\right〉 = {e}^{- {inϕ}_{{b}_{r}}} \left|n\right〉 \left|R\right〉 .$ (39)

For simplicity the state |*qⱼ*〉 is denoted as a quantum register encoding a vibrational wavefunction over the j-th vibrational mode, as shown in Fig. 6. Thus, by generating a register that encodes a particular polynomial term, e.g. Φ*ᵢⱼq̂ᵢq̂ⱼ* through the use of quantum multiplication circuits, the associated phase can be implemented by using a quantum addition circuit as $\left|{Φ}_{ij}{q}_{i}{q}_{j}\right〉 ⊕ \left|R\right〉 = {e}^{- i {Φ}_{ij} {q}_{i} {q}_{j}} \left|{Φ}_{ij}{q}_{i}{q}_{j}\right〉 \left|R\right〉 .$ (40)

A circuit for implementing this operation is presented in Fig. 11. The advantage of using this implementation for compiling the exponentials comes from the fact that quantum arithmetic routines have a cost scaling linearly with the number of qubits. For a given D-th order Taylor expansion of the PES, this approach will need D - 1 additional registers to carry the intermediate coefficients, each requiring *N · d* qubits for d = 2*, ··· , D.* Two more registers are then needed: one for encoding the monomial coefficient Φ*ᵢⱼ*, alongside another register to carry the result of multiplying this coefficient by the multiplication of q's. Finally, a resource state is required for each different Taylor expansion degree: the *ϕ_{bᵣ}* constant in each must be adjusted as to account for the Δ*^{d}* factors associated with a d-th degree monomial of *q̂ⱼ*'s such that phase gradient associated with the integer encoding Φ*ᵢⱼqᵢqⱼ* corresponds to *e*^{*i*Φ}*^{^{ij}qⁱq^{j}}.* This then entails D - 1 resource state registers, each having *bᵣ*-qubits.

In addition, intermediate coefficients can be used for implementing several different coefficients. For example, a register carrying |*qᵢqⱼ*〉 can be used for implementing both terms with *q̂ᵢq̂ⱼ* and ${\hat{q}}_{i}^{2} {\hat{q}}_{j}$*.* Thus, several coefficients can be implemented before passing to the uncomputation step that corresponds to the operations to the right of the addition gate in Fig. 11. This is referred to as caching of coefficients: on top of the high efficiency of the quantum arithmetic implementations, caching is an optimization that allows for an additional 50-70% reduction in the number of independent multiplications required to implement all the terms within a single Trotter step, depending on the particular structure of the Hamiltonian coefficients. Note that the number of additions cannot be reduced through caching, as each non-zero coefficient in the Taylor representation of the Hamiltonian in equation (4) will involve one addition.

The overall process can be benchmarked by simulating the complete workflow. PennyLane^{™} was used for simulating the quantum algorithm, obtaining vibrational Hamiltonians, and obtaining the VSCF solutions.

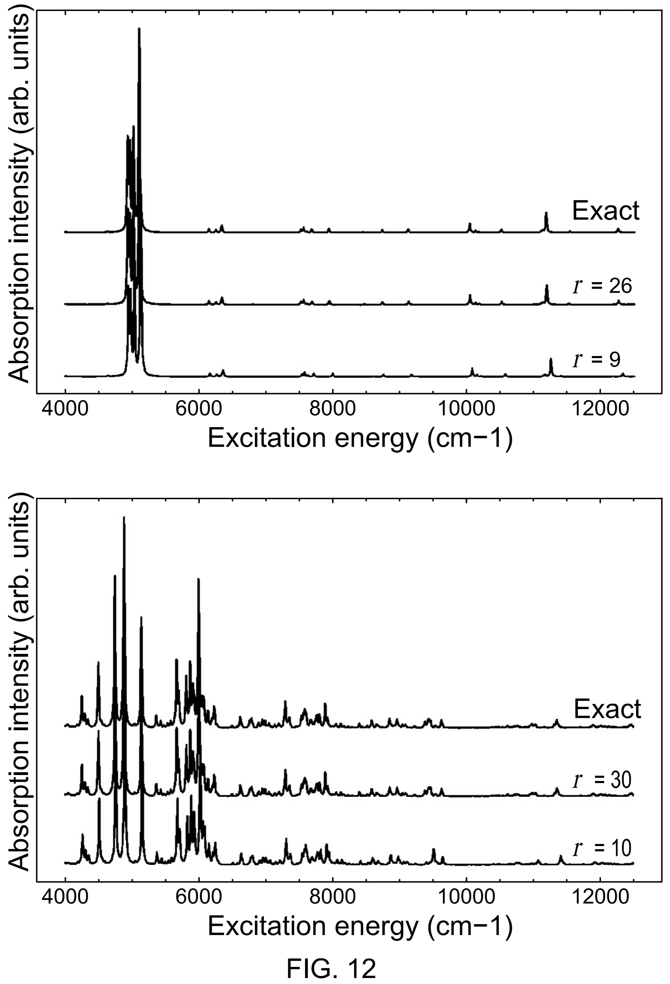

Fig. 12 shows the spectra obtained by simulating the quantum algorithm for both hydrogen sulfide H₂S and formaldehyde CH₂O molecules for different values of the Trotter step size r. Both a larger and a smaller value of r are shown in these plots: these were obtained using the perturbative approach for an error of respectively *ε_{ν}* = 1 cm⁻¹ and *ε_{ν}* = 10 cm⁻¹. For benchmarking purposes, the reference simulation results (black) are also included, obtained by exact diagonalization for the smaller H₂S molecule where it is available and large values of *r* with a converged spectrum for CH₂O where exact diagonalization was not possible. These results establish a proof-of-concept for both the spectrum simulation using the proposed quantum algorithm, as well as the perturbation theory-based choice of the Trotter step r. These results establish aproof-of-concept that the system and method described herein can recover high-quality NIR spectra as desired. Finally, it is shown how *ε_{ν}* can be adapted depending on the desired accuracy of the simulated spectrum, with the less stringent *ε_{ν}* = 10 cm ⁻¹ already yielding high-quality spectra.

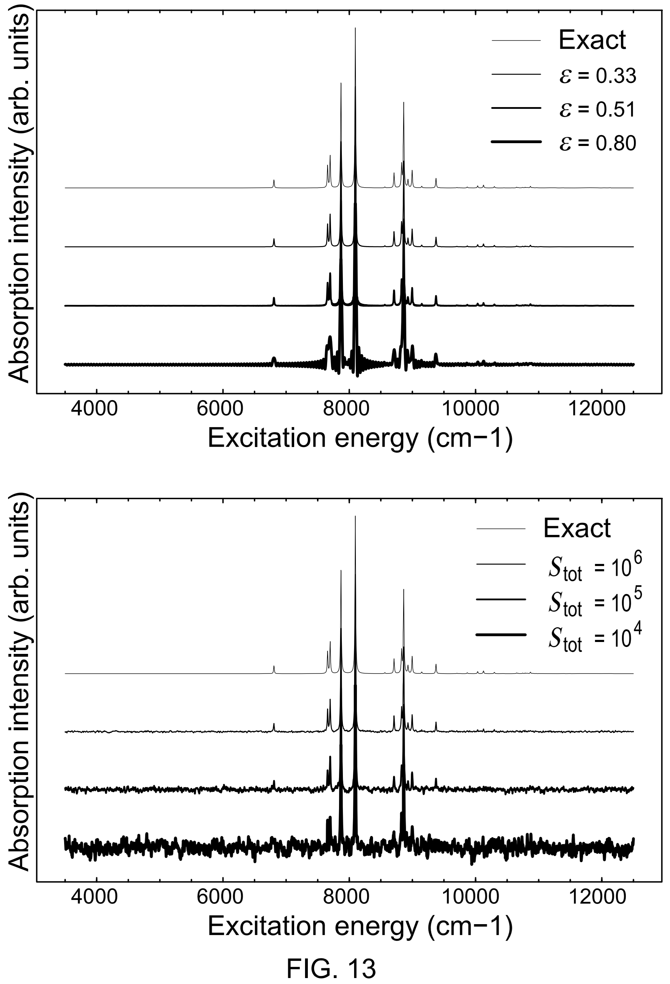

The hyperparameters in the methods described herein may be selectedbased on the discrete Fourier transform accuracy *ε* appearing in equation (14) and the total number of shots 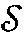 in equation (22). In one examplary embodiment, 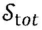 is defined as 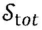 *=* 3 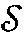 to incorporate the total number of shots across all contributions to the absorption spectrum coming from the different Cartesian components of the dipole. The choice of the hyperparameters is heuristic and depends on the level of precision required, which means that for different applications these could change. However, this may be treated purely as a signal processing problem. Both of these quantities are associated to the required accuracy of the Fourier representation for a given function with some target Lorentzian broadening and spectral range. In the same example, these are considered to be η = 10*cm*⁻¹ and Ω = 9,000*cm*⁻¹ respectively, reflecting the typical values observed in NIRS. A randomly generated NIR spectra were used for determining the hyperparameters *ε* and 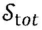 in Fig. 13. The hyperparameters are varied as to obtain what was deemed to meet the exact spectrum within a suitable degree. From this heuristic analysis, it is determined that a value of *ε* = 0.51 and of 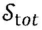 = 10⁵ is sufficient to recover high-quality spectra. Finally, it is noted that the usage of more advanced post-processing techniques, such as matching pursuit algorithms or compressed sensing, could be beneficial for lowering the required number of circuit repetitions.

The optimizations used in the overall example method are summarized in Table 1, below for the example of the azidoacetylene HC₂N₃ molecule.

**Table 1: List of optimizations with associated estimated improvement ratio to the total runtime required to simulate NIR spectra.**

| **Optimization technique** | **Additional qubits** | **Total qubits** | **Approximate improvement** | **Cumulative improvement** |
|---|---|---|---|---|
| 1 - Mode localization | 0 | 49 | × 50 | 5 × 10¹ |
| 2 - Real-space representation | 124 | 173 | × 8 | 4 × 10² |
| 3 - Coefficient caching | 0 | 173 | × 2 | 8 × 10² |
| 4 - Trotter perturbative step | 0 | 173 | × 2 | 1.6 × 10³ |
| 5 - Double measurement trick | 49 | 222 | × 1.7 | 2.7 × 10³ |
| 6 - Double phase trick | 0 | 222 | × 2 | 5.4 × 10³ |
| 7 - Initial state projection | 0 | 222 | × 1000 | 5.4 × 10⁶ |
| 8 - Active volume compilation | 278 - 1778 | 500 - 2000 | × 25 -× 100 | 1.4 × 10⁸ - 5.6 × 10⁹ |

The cumulative improvement column corresponds to an aggregate of all optimizations steps described herein on top of the associated row, while total qubits corresponds to the associated number of qubits. Numbers shown are obtained for the azidoacetylene HC₂N₃ molecule with 12 vibrational modes; some optimization steps may have a larger improvement for bigger molecules. The mode localization improvement was calculated through the change of number of terms in the Hamiltonian obtained with a 2-mode expansion instead of 3-mode. The real-space representation improvement was calculated with respect to the second-quantized modal-based representation of the Hamiltonian. The Trotter step improvement was calculated with respect to the usage of the commutator-based bounds. Two different budgets of 500 and 2000 logical qubits were considered with the active volume compilation technique to demonstrate the different improvements.

Given the method for obtaining first-order dipole spectra on a quantum computer as described herein, Table 2 below shows the associated resource estimates for a variety of molecules. As shown, the overall runtimes are in the order of a few seconds/minutes for single steps and a few hours/days for the entire flow. When also considering the circuit requirements of few hundreds of logical qubits and potentially billions of T-gates per quantum circuit, this establishes the simulation of NIR spectra as a promising application of early fault-tolerant quantum computers for going beyond current classical computing capabilities.

**Table 2: Resource estimates for overall process.**

| **Molecule** | **Vibrational modes** | **Trotter *r*** | **Maximum T-gates (runtimes in seconds)** | **Total T-gates (runtimes in hours)** |
|---|---|---|---|---|
| H₂S | 3 | 10 | 1.51 × 10⁸ (6) | 1.85 × 10¹² (21) |
| NO₂ | 3 | 3 | 4.79 × 10⁷ (2) | 6.02 × 10¹¹ (7) |
| H₂O | 3 | 14 | 2.12 × 10⁸ (9) | 2.59 × 10¹² (29) |
| CO₂ | 4 | 8 | 9.12 × 10⁷ (4) | 1.12 × 10¹² (12) |
| CH₂O | 6 | 11 | 5.48 × 10⁸ (22) | 6.68 × 10¹² (74) |
| C₂N₂ | 6 | 8 | 2.75 × 10⁸ (11) | 3.37 × 10¹² (37) |
| CH₄ | 9 | 13 | 1.80 × 10⁹ (72) | 2.19 × 10¹³ (243) |
| C₂H₄ | 12 | 13 | 2.36 × 10⁹ (94) | 2.87 × 10¹³ (319) |
| HC₂N₃ | 12 | 6 | 1.52 × 10⁹ (61) | 1.87 × 10¹³ (208) |

In Table 2, the maximum T-gates column represents the number of T-gates associated with the deepest circuit in the implementation of the method as described herein, i.e. derived from the maximum time *k*ₘ*ₐₓ,* with total T-gates considering all the circuit repetitions for reconstructing the spectrum. Associated runtimes are shown in parenthesis, which are determined using the active volume compilation technique from with a clock-rate of *ν*_{c*lock*} = 1 MHz and a budget of 500 logical qubits. A basis of 4 qubits was used for discretizing each vibrational mode, alongside a value of *ε_{ν}* = 10 cm ⁻¹ for the perturbative of the Trotter step and a total of 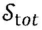 *=* 10⁵ shots distributed across all different times and Cartesian components of the dipole.

As evoked above, the spectrum acquired using embodiments of processes such as described herein can constitute synthetic data which can be used as a reference to which spectrum acquired from a sample can be compared. In practice, typical embodiments would make such a direct comparison between a measurement taken from a sample and the synthetic data difficult using traditional algorithms.

An alternative to using synthetic data is to use experimental data. For instance, a trained statistical model could be used to detect a given molecule using its spectrum. Training statistical models requires large amounts of data to achieve high accuracy and transferability between measurement apparatuses and experimental contexts. Collecting enough data for model training is often expensive or otherwise challenging. This could be due to working with dangerous chemicals (e.g. toxic or explosive), the conformers being hard to separate in the laboratory, or simply due to the large amount of data needed in the first place, for example when working with natural products.

Simulations of NIR spectra using a quantum computer, using embodiments such as presented herein, providing a source of "synthetic data", may help address these limitations. First, augmenting experimental datasets with simulated data, or even replacing experimental datasets with simulated data, could significantly alleviate the costs associated with data acquisition, and speed up the deployment process. Simulated data are also inherently safe to procure, removing any security concerns. Second, simulations provide additional information above and beyond the spectrum itself, for instance access to the underlying states and their symmetries, ability to vary geometry, environment effects, and so on. This information may be used to improve the sensitivity and specificity of statistical models, for example by selecting the key wavelengths most correlated with the property of interest on physical grounds, rather than purely statistically. And finally, this same information may also be used to aid in interpreting the results of a statistical model, building spectrum-structure relationships, thus enhancing reliability, trust, and transferability of the model.

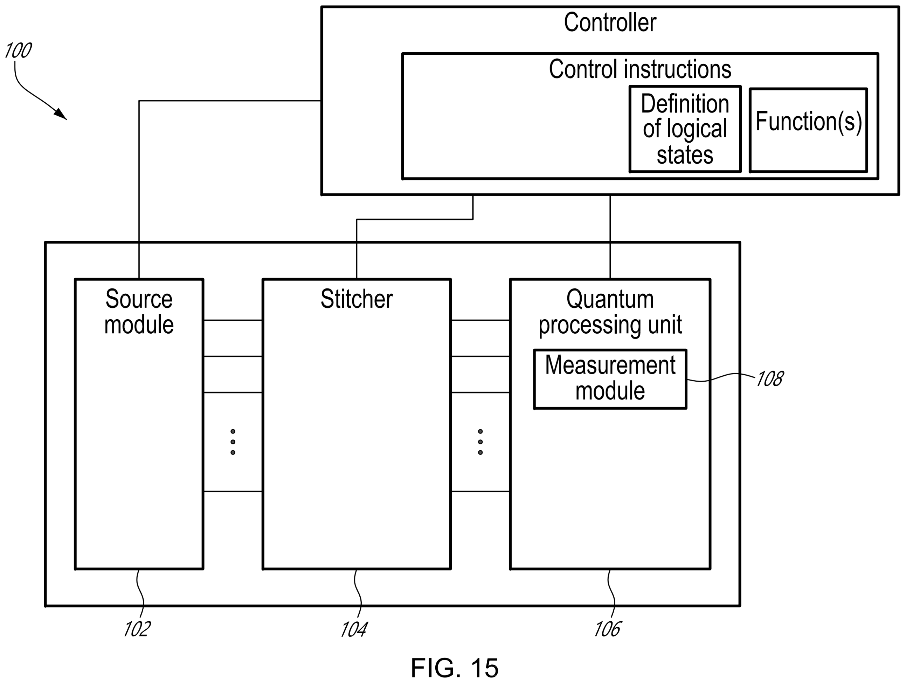

Fig. 15 illustrates a simplified system-level block diagram of another example of a quantum computer 100 which can be used to implement the processes described above. In this example, the quantum computer 100 is based on a photonic architecture and uses principles of measurement-based quantum computing (MBQC) and offers a contrast to the example quantum computer 40 presented in Fig. 1, and which used principles of gate-based quantum computing. The components of the quantum computer 100, described in more detail below, can be configured to one or more of: create, generate, shape, measure, decode, and error-correct a selected entangled cluster state that serves as a resource for measurement-based fault-tolerant quantum computation. For example, the quantum computer 100 may be a measurement-based quantum computer in the Raussendorf-Harrington-Goyal (RHG) model using continuous-variable (CV) qubits. Although an example is described below in the context of a measurement-based quantum computing model based on a photonic platform, it is understood that embodiments of the methods described herein are also applicable to other quantum platforms, including superconducting qubits, trapped ion qubits, and any other quantum platforms, and may be of use in circuit-based quantum computing models and even potentially in adiabatic quantum computing models.

Returning to the example presented in Fig. 15, in some embodiments, the quantum computer 100 includes a source module 102, a stitcher 104, and a quantum processing unit (QPU) 106. Each of the source module 102, the stitcher 104, and the QPU 106 represents logical functionality that can be implemented exclusively via hardware, exclusively via software, or via a combination of hardware and software. The functional delineation between the separate elements within the quantum computer 100 may only be a conceptual representation of the overall operation of the quantum computer 100.

The source module 102 is configured to generate a plurality of input quantum states. In the case of photonic quantum platforms, the source module 102 may include a plurality of Gaussian Boson Sampling (GBS) sources each configured to generate a continuous-variable (CV) state of light, such as Gottesman-Kitaev-Preskill (GKP) state. Data is mapped onto the generated CV states.

In some embodiments, the source module 102 may include a local pulse train generator. The local pulse train generator is configured to generate a train of local optical pulses that are also referred to as a local oscillator (LO) signal that is operably coupled to elements of quantum computer 100, such as QPU 106, and particularly to the optical detectors (e.g., homodyne detectors) used for projective measurements and for measurement-based quantum computation.

A steady supply of CV states that include both encoded bosonic qubits and squeezed vacuum states may be generated by the source module 102 and sent to the stitcher 104 via a network of optical connections. In some embodiments, the source module 102, the stitcher 104, and the QPU 106 are implemented on photonic integrated circuits (PICs), and the optical network may be implemented using optical fibers compatible with existing optical technologies, which may have a lower level of propagation loss compared to on-chip waveguides. In some embodiments, the source module 102 generates the desired CV states by performing Pauli measurements on intermediary CV states within the source module 102. In the case of photonic quantum computing architectures, the Pauli measurements may be performed by way of homodyne detection.

The states generated by the source module 102 are provided to the stitcher 104, which is configured to stitch the input states into a lattice structure in accordance with a specific encoding scheme. In some embodiments, the stitcher 104 can interfere these states, such as through a network of static 50:50 beamsplitters with phase delays or reconfigurable beamsplitters into what is referred to as a higher dimensional multimode entangled state, referred to hereinbelow as a cluster state for short, before sending/routing the multimode entangled state to the QPU 106 to be processed. As used herein, "stitching" refers to the creation/imposition of entanglement between the different modes at different lattice sites. In some embodiments, during operation of the quantum computer 100, non-deterministically generated encoded qubits and non-Gaussian states of light can be stitched into a random but known subset of the sites (by virtue of them being generated at a random subset of locations, and not being generated at other locations), while the remaining sites are populated with deterministically generated Gaussian states, such as squeezed vacuum states for example. An indication of whether a location is within the subset of the sites is provided.

Measurements can be performed on the higher dimensional multimode entangled state by a measurement module 108 of the QPU 106 to provide electric field quadrature information (e.g., the position or amplitude quadrature referred to herein as the "q quadrature" and the momentum or phase quadrature referred to herein as the "*p* quadrature"). The measurements are performed by one or more optical detectors of the measurement module 108. In some embodiments, the optical detectors are homodyne detectors which can be used to perform measurements on each macronode of the multimode state to reduce each macronode into a single node with multiple edges. The measurement outcomes can be used by the quantum computing system to perform error correction and can also be utilized by the system to perform measurement-based quantum computation (MBQC). The optical detectors, (e.g., homodyne detectors) may be configured to perform quadrature measurements by interfering the optical modes of an input entangled resource state and the train of local optical pulses on a beamsplitter and detecting the optical power difference of the two beam splitter outputs as an indication of the modal property(ies) of the quantum state of the given optical pulse. The measurement outcomes collected on the multimode entangled states (i.e., at the physical hardware layer) can be processed together to implement one or more aspects of an error correction code. The measurement results can be processed by the QPU 106, which may be configured to take, as inputs, the measurement outcomes that have been realized to perform the logical operations.

The control of the at least one of and typically each one of the source module 102, stitcher 104, and quantum processing unit 106 cannot be performed manually and is performed via hardware and software elements which will be referred to herein as a controller. The controller can be a classical computer, i.e., a computer based on bits rather than qubits for computation, including at least one processor wherein control instructions are stored in computer-readable memory and are accessible to the at least one processor. The control instructions can include a plurality of functions, such as a source module control function, a stitcher module control function, and a quantum processing unit control function, each of which can include a plurality of sub-processes. The control instructions typically include a definition of logical states which are mapped to the specific hardware elements of the given embodiment.

It will be understood that the expression "classical computer" as used herein, is not to be interpreted in a limiting manner. It is rather used in a broad sense to generally refer to the combination of some form of one or more processing units and some form of memory system accessible by the processing unit(s). The memory system can be of the non-transitory type. The use of the expression "computer" in its singular form as used herein includes within its scope the combination of two or more computers working collaboratively to perform a given function. Moreover, the expression "computer" as used herein includes within its scope the use of partial capabilities of a given processing unit. Example computers include supercomputers, desktops, laptops, smartphones, smart watches, less elaborated controller devices, etc.

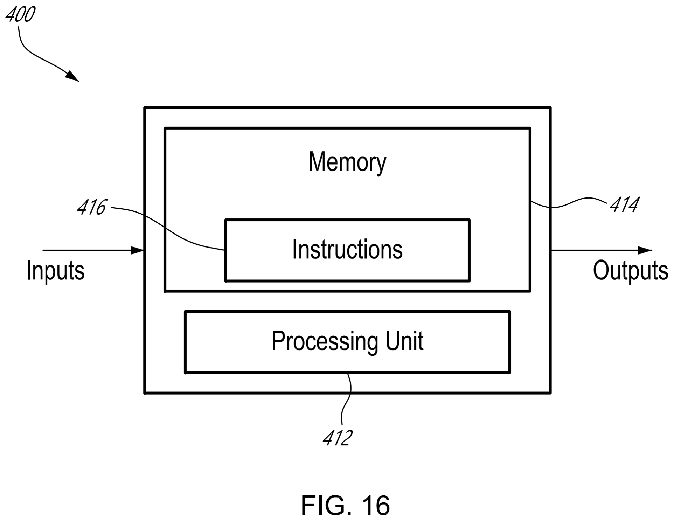

An example classical computer 400 is presented in Fig. 16. In this example, the computer 400 includes a processing unit 412 and a memory system 414 storing instructions 416.

A processing unit 412 can be embodied in the form of a general-purpose microprocessor or microcontroller, a digital signal processing (DSP) processor, an integrated circuit, a field programmable gate array (FPGA), a reconfigurable processor, or a programmable read-only memory (PROM), to name a few examples.

The memory system 414 can include a suitable combination of any suitable type of computer-readable memory located either internally or externally, and accessible by the processor in a wired or wireless manner, either directly or over a network such as the Internet. A computer-readable memory can be embodied in the form of random-access memory (RAM), read-only memory (ROM), compact disc read-only memory (CDROM), electro-optical memory, magneto-optical memory, erasable programmable read-only memory (EPROM), electrically erasable programmable read-only memory (EEPROM), or ferroelectric RAM (FRAM), to name a few examples.

A computer 400 can have one or more input/output (I/O) interfaces to allow communication with a human user and/or with another computer via an associated input, output, or input/output device such as a keyboard, a mouse, a touchscreen, an antenna, a port, etc. Each I/O interface can enable the computer to communicate and/or exchange data with other components, to access and connect to network resources, to serve applications, and/or to perform other computing applications by connecting to a network (or multiple networks) capable of carrying data including the Internet, Ethernet, plain old telephone service (POTS) line, public switch telephone network (PSTN), integrated services digital network (ISDN), digital subscriber line (DSL), coaxial cable, fiber optics, satellite, mobile, wireless (e.g., Wi-Fi, Bluetooth, WiMAX), SS7 signaling network, fixed line, local area network, or wide area network, to name a few examples.

It will be understood that a computer can perform functions or processes via hardware or a combination of both hardware and software. For example, hardware can include logic gates included as part of a silicon chip of a processor. Software (e.g., an application or process) can be in the form of data such as computer-readable instructions stored in a non-transitory computer-readable memory accessible by one or more processing units. With respect to a computer or a processing unit, the expression "configured to" relates to the presence of hardware or a combination of hardware and software which is operable to perform the associated functions. Different elements of a computer, such as processor and/or memory, can be local, in part remote, or in whole remote and/or distributed and/or virtual.

The methods and systems performed by a classical computer in the context of the present disclosure may be implemented in a high-level procedural or object-oriented programming or scripting language, or a combination thereof, to communicate with or assist in the operation of a computer system, for example the controller. Alternatively, the methods and systems described herein may be implemented in assembly or machine language. The language may be a compiled or interpreted language. Program code for implementing the methods and systems described herein may be stored on a storage media or a device, for example a ROM, a magnetic disk, an optical disc, a flash drive, or any other suitable storage media or device. The program code may be readable by a general or special-purpose programmable computer for configuring and operating the computer when the storage media or device is read by the computer to perform the procedures described herein. Embodiments of the methods and systems described herein may also be considered to be implemented by way of a non-transitory computer-readable storage medium having a computer program stored thereon. The computer program may comprise computer-readable instructions which cause a computer, or more specifically the processing unit of the computing device, to operate in a specific and predefined manner to perform the functions described herein.

Computer-executable instructions may be in many forms, including program modules, executed by one or more computers or other devices. Generally, program modules include routines, programs, objects, components, data structures, etc., that perform particular tasks or implement particular abstract data types. Typically, the functionality of the program modules may be combined or distributed as desired in various embodiments. The technical solution of embodiments may be in the form of a software product. The software product may be stored in a non-volatile or non-transitory storage medium, which can be a compact disk read-only memory (CDROM), a USB flash disk, or a removable hard disk. The software product includes several instructions that enable a computer device (personal computer, server, or network device) to execute the methods provided by the embodiments.

The embodiments described herein are implemented by physical computer hardware. The embodiments described herein provide useful physical machines and particularly configured computer hardware arrangements. The embodiments described herein are directed to electronic and optical machines and methods implemented by electronic and optical machines adapted for processing and transforming electromagnetic signals which represent various types of information. The embodiments described herein pervasively and integrally relate to machines, and their uses; and the embodiments described herein have no meaning or practical applicability outside their use with computer hardware, machines, and various hardware components. Substituting the physical hardware particularly configured to implement various acts for non-physical hardware, using mental steps for example, may substantially affect the way the embodiments work. Such computer hardware limitations are clearly essential elements of the embodiments described herein, and they cannot be omitted or substituted for mental means without having a material effect on the operation and structure of the embodiments described herein. The computer hardware is essential to implement the various embodiments described herein and is not merely used to perform steps expeditiously and in an efficient manner.

As can be understood, the examples described above and illustrated are intended to be exemplary only. The scope is indicated by the appended claims.

## Claims

1. A method of determining a spectrum stemming from an electromagnetic interaction with one or more molecules, the method comprising:
performing a plurality of iterations including
preparing an initial state of a Hamiltonian acted upon by a dipole operator in a system register of a quantum computer, the Hamiltonian encoding the one or more molecules and the dipole operator encoding the electromagnetic interaction in a target orientation,
encoding information pertaining to a distribution of possible values of a delay for a time evolution into one or more auxiliary qubits of the quantum computer, and
encoding information pertaining to a product of the distribution with one or more expectation values into the quantum computer;
computing a leading-order error operator based on one or more diagonalizable fragments of the Hamiltonian and a product formula approximating the time evolution;
computing a set of eigenstates representing a classical solution of the Hamiltonian;
computing product formula expectation values of the leading-order error operator for at least some eigenstates of the set of eigenstates;
computing a maximum time step associated with a target error using the product formula expectation values;
performing one or more Fourier transforms of the product of the distribution with the one or more expectation values in a discretized manner, over one or more time steps, the one or more time steps being equal to or lesser than the maximum time step; and
determining the spectrum based on the one or more Fourier transforms.

2. The method of claim 1 further comprising representing the initial state as a linear combination of eigenstates of the set of eigenstateswherein said computing the leading-order error operator preferably includes performing a Baker-Campbell-Hausdorf (BCH) expansion, further comprising:
determining eigenstates of interest having significant overlap with the initial state based on the linear combination of the eigenstates;
computing a weighted average of individual maximum time steps associated to different ones of the eigenstates of interest; and
determining the maximum time step based on weighted average.

3. The method of claim 2, further comprising
identifying, from the linear combination eigenstates, eigenstates which are outside a window of frequencies of interest for the spectrum; and
removing, from the linear combination of eigenstates, eigenstates which are outside the window of frequencies of interest for the spectrum, wherein said removing preferably includes building a projection operator and applying the projection operator onto the linear combination of eigenstates.

4. The method of claim 3 wherein said preparing the initial state of the Hamiltonian is based on the linear combination of eigenstates subsequently to said removing.

5. The method of any one of claim 3 or 4 further comprising: determining a number of wavenumbers between eigenvalues of an approximate solution and eigenvalues of an exact solution, calculating a buffer based on the number of wavenumbers, and factoring in the buffer into the window of frequencies.

6. The method of any one of claims 1 to 5 wherein the time evolution is between the initial state and a final state, the final state corresponding to a time at which expectation values of the dipole operator in a final orientation are measured, wherein the steps of performing a plurality of iterations and performing the one or more Fourier transform are repeated for more than one target orientation with the final orientation corresponding to the target orientation, wherein the repeating is preferably performed for three orientations of a coordinate system corresponding to on-diagonals of a 3 by 3 matrix.

7. The method of any one of claims 1 to 6 wherein the dipole operator corresponds to linear absorption spectroscopy.

8. The method of claim 6 or 7 wherein the steps of performing a plurality of iterations and performing the one or more Fourier transform are further repeated for more than one combination of the final orientation and the target orientation, with the final orientation different from the target orientation, wherein the further repeating is preferably performed for three orientations of a coordinate system corresponding to off-diagonals of a 3 by 3 matrix, and wherein the dipole operator preferably corresponds to angular resolved spectroscopy.

9. The method of any one of claims 1 to 8 wherein said encoding the information pertaining to the product includes applying time-evolution operators of the form *e^{-iHδt}* to the system register, controlled on the one or more auxiliary qubits, where H is the Hamiltonian and *δt* is one or more of the possible values, wherein the time steps form corresponding portions of *δt*.

10. The method of any one of claims 1 to 9 wherein for each iteration:
said encoding information pertaining to the distribution includes encoding the distribution of all possible values of the delay as an initial superposition into more than one of the one or more auxiliary qubits forming an auxiliary register of the quantum computer;
said encoding information pertaining to the product includes applying the time evolution on the system register, multiplexed on the auxiliary register
said performing the one of the one or more Fourier transform includes applying a quantum Fourier transform to the auxiliary register, subsequently to said applying the time-evolution; and
a measurement is performed of the auxiliary register subsequently to said applying one of the one or more Fourier transform, including sampling a frequency of the spectrum;
further comprising building one or more histograms based on the measurements acquired from each one of the plurality of iterations, and obtaining the response spectrum from the one or more histograms, wherein each iteration preferably further includes determining whether a measurement of the system register corresponds to the initial state, wherein said building the histogram includes integrating the measurement of the auxiliary register to the histogram contingent upon said measurement of the system register corresponding to the initial state.

11. The method of claim 10 wherein said building the histogram includes normalizing the histogram based on the measurements acquired in each one of the iterations.

12. The method of claim 10 or 11 wherein each iteration further includes applying block encoding of the dipole in the target direction, applying a Hermitian conjugate of a preparation circuit to the system register, and measuring the system register, between said applying the time evolution and applying the Fourier transform.

13. The method of any one of claims 1 to 9 wherein each iteration further includes:
isolating one of said possible values, including sampling the distribution;
performing a Hadamard test on the isolated value including applying a first Hadamard gate to one of the auxiliary qubits forming an ancilla qubit, thereby preparing the ancilla qubit into a superposition, applying said time evolution controlled on the ancilla qubit, and performing a measurement yielding a single random variable encoding a component of the product associated to the isolated value; and
further comprising building a time series of the single random variables obtained from the plurality of iterations, and said performing one or more Fourier transforms includes computing a Fourier transform classically on the time series, preferably further comprising repeating said performing the Hadamard test including applying a Hermitian conjugate of a phase gate on the ancilla qubit, applying a second Hadamard gate on the ancilla qubit and measuring the ancilla qubit, preferably further comprising prepare an additional register with a state, and following measuring the ancilla qubit, performing a SWAP test between the additional register and the system register controlled by the ancilla qubit, and modifying the single random variables based on the output of the SWAP test.

14. A method of determining a spectrum stemming from an electromagnetic interaction with one or more molecules, the method comprising:
performing a plurality of iterations including
preparing an initial state of a Hamiltonian acted upon by a dipole operator in a system register of a quantum computer, the Hamiltonian encoding the one or more molecules and the dipole operator encoding the electromagnetic interaction in a target orientation,
encoding information pertaining to a distribution of possible values of a delay for a time evolution into one or more auxiliary qubits of the quantum computer, and
encoding information pertaining to a product of the distribution with one or more expectation values into the quantum computer;
computing a leading-order error operator based on one or more diagonalizable fragments of the Hamiltonian and a product formula approximating the time evolution;
computing a set of eigenstates representing a classical solution of the Hamiltonian;
computing product formula expectation values of the leading-order error operator for at least some eigenstates of the set of eigenstates;
computing a maximum time step associated with a target error using the product formula expectation values;
performing one or more Fourier transforms of the product of the distribution with the one or more expectation values in a discretized manner, over one or more time steps, the one or more time steps being equal to or lesser than the maximum time step; and
determining the spectrum based on the one or more Fourier transforms.

15. The method of any one of claims 1 to 9 wherein for each iteration:
said encoding information pertaining to the distribution includes encoding the distribution of all possible values of the delay as an initial superposition into more than one of the one or more auxiliary qubits forming an auxiliary register of the quantum computer;
said encoding information pertaining to the product includes applying the time evolution on the system register, multiplexed on the auxiliary register
said performing the one of the one or more Fourier transform includes applying a quantum Fourier transform to the auxiliary register, subsequently to said applying the time-evolution; and
a measurement is performed of the auxiliary register subsequently to said applying one of the one or more Fourier transform, including sampling a frequency of the spectrum;
further comprising building one or more histograms based on the measurements acquired from each one of the plurality of iterations, and obtaining the response spectrum from the one or more histograms, wherein each iteration preferably further includes determining whether a measurement of the system register corresponds to the initial state, wherein said building the histogram includes integrating the measurement of the auxiliary register to the histogram contingent upon said measurement of the system register corresponding to the initial state.

16. The method of any one of claims 1 to 9 wherein each iteration further includes:
isolating one of said possible values, including sampling the distribution;
performing a Hadamard test on the isolated value including applying a first Hadamard gate to one of the auxiliary qubits forming an ancilla qubit, thereby preparing the ancilla qubit into a superposition, applying said time evolution controlled on the ancilla qubit, and performing a measurement yielding a single random variable encoding a component of the product associated to the isolated value; and
further comprising building a time series of the single random variables obtained from the plurality of iterations, and said performing one or more Fourier transforms includes computing a Fourier transform classically on the time series.

17. The method of claim 19 further comprising repeating said performing the Hadamard test including applying a Hermitian conjugate of a phase gate on the ancilla qubit, applying a second Hadamard gate on the ancilla qubit and measuring the ancilla qubit, preferably further comprising prepare an additional register with a state, and following measuring the ancilla qubit, performing a SWAP test between the additional register and the system register controlled by the ancilla qubit, and modifying the single random variables based on the output of the SWAP test.

18. A non-transitory computer-readable storage medium having stored thereon instructions which, when executed upon by a computing device, cause the computing device to perform a method comprising:
performing a plurality of iterations, each iteration including
preparing an initial state of a Hamiltonian acted upon by a dipole operator in a system register of a quantum computer, the Hamiltonian encoding one or more molecules and the dipole operator encoding an electromagnetic interaction with the one or more molecules in a target orientation,
encoding information pertaining to a distribution of possible values of a delay for a time evolution into one or more auxiliary qubits of the quantum computer, and
encoding information pertaining to a product of the distribution with one or more expectation values into the quantum computer;
computing a leading-order error operator based on one or more diagonalizable fragments of the Hamiltonian and a product formula approximating the time evolution;
computing a set of eigenstates representing a classical solution of the Hamiltonian;
computing product formula expectation values of the leading-order error operator for at least some eigenstates of the set of eigenstates;
computing a maximum time step associated with a target error using the product formula expectation values;
performing one or more Fourier transforms of the product of the distribution with the one or more expectation values in a discretized manner, over one or more time steps, the one or more time steps being equal to or lesser than the maximum time step; and
determining a spectrum based on the one or more Fourier transforms.
